# EUROPEAN PATENT APPLICATION

(11) **EP 4 831 302 A1**
(43) Date of publication of application: **28.10.2026**
(21) Application number: 26174306.6
(22) Date of filing: 23.04.2026
(51) Int. Cl.: C12Q 1/70, C12Q 1/6883

(54) **COMPOSITIONS AND METHODS FOR DETECTING SEVERE FEVER AND THROMBOCYTOPENIA SYNDROME VIRUS**

(30) Priority: 23.04.2025 US 202563793084 P
(71) Applicant: Abbott Laboratories, Abbott Park, IL 60064 (US)
(72) Inventor: Hodges, Austin, Abbott Park (US); Berg, Michael, Abbott Park (US); Perez, Lester, Abbott Park (US)
(74) Representative: Mathys & Squire

(57) **Abstract**

Provided herein are compositions, methods, and kits for detecting *Severe fever with thrombocytopenia syndrome virus* (SFTSV) in human subjects. In certain embodiments, provided herein are SFTSV specific nucleic acid probes and primers, and methods for detecting SFTSV nucleic acid in human subjects.

## Description

### CROSS-REFERENCE TO RELATED APPLICATIONS

This application claims the benefit of U.S. Provisional Application No. 63/793,084, filed April 23, 2025, the content of which is herein incorporated by reference in its entirety.

### SEQUENCE LISTING STATEMENT

The contents of the electronic sequence listing titled ABBTL_44791_202_SequenceListing.xml (Size: 16,589 bytes; and Date of Creation: April 13, 2026) is herein incorporated by reference in its entirety.

### TECHNICAL FIELD

Provided herein are compositions, methods, and kits for detecting *Severe fever with thrombocytopenia syndrome virus* (SFTSV) in human subjects. In certain embodiments, provided herein are SFTSV specific nucleic acid probes and primers, and methods for detecting SFTSV nucleic acid in human subjects.

### BACKGROUND

*Severe Fever with Thrombocytopenia Syndrome Virus* (SFTSV) is an emerging tick-borne pathogen (Casel, Park and Choi 2021) that poses a significant public health threat (Kim and Park 2023). Although SFTSV infection can present severe clinical symptoms, including prolonged high fever (Luo, Li et al. 2023), thrombocytopenia, gastrointestinal distress (Akagi, Miyazaki et al. 2020), and in critical cases, progression to multi-organ failure (Li, Li et al. 2018), a substantial proportion of infections are asymptomatic or exhibit only mild, non-specific symptoms (Huang, Jiang et al. 2017). These subclinical or mildly symptomatic individuals can act as silent carriers (Wang, Deng et al. 2014), allowing the virus to spread unnoticed within communities (Xu, Xue et al. 2024). Stealth transmission can indeed complicate efforts to detect and control outbreaks (Shaikh, Swali and Houben 2023) as the virus disseminates without obvious clinical signs, hindering timely diagnosis and intervention.

Since its initial identification in China in 2009 (Yu, Liang et al. 2011), SFTSV has been reported in several countries across Asia and neighboring regions. Cases have emerged in South Korea in 2012 (Kim, Yi et al. 2013), Japan in 2013 (Takahashi, Maeda et al. 2013), Vietnam, Taiwan, Myanmar and Thailand in 2019 (Tran, Yun et al. 2019, Peng, Yang et al. 2020, Win, Nguyen et al. 2020, Rattanakomol, Khongwichit et al. 2022), and Pakistan in 2020 (Zohaib, Zhang et al. 2020). This pattern of geographic spread has been associated with the expansion of one of its primary vectors (Casel, Park and Choi 2021, Sato, Mekata et al. 2021), the invasive tick species Haemaphysalis longicornis. The dispersal of this tick species into new territories may have facilitated the transmission of SFTSV, contributing to its increasing incidence and wider distribution (Pérez, Baele et al. 2024).

Thus, there is need for compositions, methods, and kits for detecting SFTSV and diagnosing SFTSV in human subjects.

### SUMMARY

Embodiments of the disclosure provide compositions and methods for detecting SFTSV in a sample from a subject. Aspects of the disclosure utilize a multi-target and multi-lineage amplification assay that detects both current and emergent strains of SFTSV. Among other uses, the compositions and methods improve patient care by differentiating SFTSV from other febrile illnesses.

In one embodiment, the present disclosure relates to a composition comprising at least two sets of primers and at least two probes for detecting *Severe fever with thrombocytopenia syndrome virus* (SFTSV) in a sample obtained from a subject, wherein the composition comprises: a first set of primers and a first probe, wherein each primer comprises a region that corresponds to a portion of a L segment of the SFTSV, and further wherein the first set of primers comprise (i) a forward primer comprising or consisting of an oligonucleotide sequence of SEQ ID NO: 1 or a complement thereof, and (ii) a reverse primer comprising or consisting of an oligonucleotide sequence of SEQ ID NO: 2 and/or 3 or a complement thereof; and the first probe comprises or consists of an oligonucleotide sequence of SEQ ID NO: 4 and/or 5 or a complement thereof; and a second set of primers and a second probe, wherein each primer comprises a region that corresponds to an S segment of the SFTSV, and further wherein the second set of primers comprise (i) a forward primer comprising or consisting of an oligonucleotide sequence of SEQ ID NO: 6 or a complement thereof, and (ii) a reverse primer comprising or consisting of an oligonucleotide sequence of SEQ ID NO: 7 or a complement thereof; and the second probe comprises or consists of an oligonucleotide of SEQ ID NO: 8 or a complement thereof; and/or a third set of primers and a third probe, wherein each primer comprises a region that corresponds to an S segment of the SFTSV, and further wherein the third set of primers comprise (i) a forward primer comprising or consisting of an oligonucleotide sequence of SEQ ID NO: 9 or a complement thereof, and (ii) a reverse primer comprising or consisting of an oligonucleotide sequence of SEQ ID NO: 10 or a complement thereof; and the third probe comprises or consists of an oligonucleotide of SEQ ID NO: 11 and/or 12 or a complement thereof.

In some aspects, the composition comprises the first and second sets of primers and probes, the first and third sets of primers and probes; or the first, second, and third sets of primers and probes.

In certain aspects (a) in the first set of primers and the first probe: (i) the forward primer further comprises a detectable label; (ii) the reverse primer further comprises a detectable label; (iii) the first probe further comprises a detectable label; or (iv) any combination of (i)-(iii); (b) in the second sets of primers and the second probes: (i) the forward primer further comprises a detectable label; (ii) the reverse primer further comprises a detectable label; (iii) the second probe further comprises a detectable label; or (iv) any combination of (i)-(iii); (c) in the third set of primers and the third probe: (i) the forward primer further comprises a detectable label; (ii) the reverse primer further comprises a detectable label; (iii) the second probe further comprises a detectable label; or (iv) any combination of (i)-(iii); or (d) any combination of (a)-(c).

In some aspects, the first, second, and third probes comprise a detectable label. In some aspects, the second and third probes comprise the same label and the first probe comprises a different label than the second and third probes.

The present disclosure is not limited to particular detectable labels. In some aspects, the first, second, and third probes comprise a quencher (e.g., at the 3' end of the probe) and a label (e.g., at the 5' end of the probe), although the locations of the quencher and the label can be reversed. Exemplary quenchers include but are not limited to tramethylrhodamine (TAMRA) or black hole quencher. Exemplary labels (e.g., fluorophores) include but are not limited to carboxyfluorescein (FAM), tetrachlorofluorescein (TET), cyanine 5 (Cy5), or hexachlorofluorescein (HEX).

In further aspects, the present disclosure provides a kit for detecting SFTSV in sample obtained from a subject, the kit comprising: a) a first set of primers and a first probe, wherein each primer comprises a region that corresponds to a portion of a L segment of the SFTSV, and further wherein the first set of primers comprise (i) a forward primer comprising or consisting of an oligonucleotide sequence of SEQ ID NO: 1 or a complement thereof, and (ii) a reverse primer comprising or consisting of an oligonucleotide sequence of SEQ ID NO: 2 and/or 3 or a complement thereof; and the first probe comprises or consists of an oligonucleotide sequence of SEQ ID NO: 4 and/or 5 or a complement thereof; and b) a second set of primers and a second probe, wherein each primer comprises a region that corresponds to an S segment of the SFTSV, and further wherein the second set of primers comprise (i) a forward primer comprising or consisting of an oligonucleotide sequence of SEQ ID NO: 6 or a complement thereof, and (ii) a reverse primer comprising or consisting of an oligonucleotide sequence of SEQ ID NO: 7 or a complement thereof; and the second probe comprises or consists of an oligonucleotide of SEQ ID NO: 8 or a complement thereof; and/or c) a third set of primers and a third probe, wherein each primer comprises a region that corresponds to an S segment of the SFTSV, and further wherein the third set of primers comprise (i) a forward primer comprising or consisting of an oligonucleotide sequence of SEQ ID NO: 9 or a complement thereof, and (ii) a reverse primer comprising or consisting of an oligonucleotide sequence of SEQ ID NO: 10 or a complement thereof; and the third probe comprises or consists of an oligonucleotide of SEQ ID NO: 11 and/or 12 or a complement thereof.

In some aspects, the kit further comprises reagents (e.g., including but not limited to, dNTPs, polymerases, nucleases, or buffers) and/or instructions for using the kit.

Additional aspects provide method of detecting SFTSV in sample obtained from a subject, wherein the method comprises the steps of: a) performing a first amplification and detection reaction with a first set of primers and a first probe, wherein each primer comprises a region that corresponds to a portion of a L segment of the SFTSV, and further wherein the first set of primers comprise (i) a forward primer comprising or consisting of an oligonucleotide sequence of SEQ ID NO: 1 or a complement thereof, and (ii) a reverse primer comprising or consisting of an oligonucleotide sequence of SEQ ID NO: 2 and/or 3 or a complement thereof; and the first probe comprises or consists of an oligonucleotide sequence of SEQ ID NO: 4 and/or 5 or a complement thereof, wherein the first amplification and detection reaction generates a first amplified product when an SFTSV L segment is present in the sample; and b) performing a second amplification and detection reaction with a second set of primers and a second probe, wherein each primer comprises a region that corresponds to an S segment of the SFTSV, and further wherein the second set of primers comprise (i) a forward primer comprising or consisting of an oligonucleotide sequence of SEQ ID NO: 6 or a complement thereof, and (ii) a reverse primer comprising or consisting of an oligonucleotide sequence of SEQ ID NO: 7 or a complement thereof; and the second probe comprises or consists of an oligonucleotide of SEQ ID NO: 8 or a complement thereof, wherein the second amplification and detection reaction generates a second amplified product when an SFTSV S segment is present in the sample; and/or c) performing a third amplification and detection reaction with a third set of primers and a third probe, wherein each primer comprises a region that corresponds to an S segment of the SFTSV, and further wherein the third set of primers comprise (i) a forward primer comprising or consisting of an oligonucleotide sequence of SEQ ID NO: 9 or a complement thereof, and (ii) a reverse primer comprising or consisting of an oligonucleotide sequence of SEQ ID NO: 10 or a complement thereof; and the third probe comprises or consists of an oligonucleotide of SEQ ID NO:11 and/or 12 or a complement thereof, wherein the third amplification and detection reaction generates a third amplified product when an SFTSV S segment is present in the sample, and detecting the presence or absence of the first, second, and/or third amplified products, wherein the presence of at least the first amplified product and the second or third amplified products is indicative of the presence of SFTVS in the sample.

In certain aspects, the first, second and third amplification and detection reactions further comprise a reverse transcription step.

In some aspects, the first, second and third amplification and detection reactions are real time PCR or real time reverse-transcriptase PCR reactions (e.g., quantitative real time PCR or quantitative real time reverse-transcriptase PCR reactions).

In certain aspects of the disclosure, the first, second and third amplification and detection reactions comprise a probe displacement and dequenching step.

The disclosure provides both automated and manual methods of amplification and detection.

Aspects of the disclosure provide a method that include a sample processing step (e.g., automated or manual sample processing).

The present disclosure is not limited to particular sample type. Examples include but are not limited to a blood sample, a plasma sample, and a serum sample.

In some aspects, the subject is a human subject.

The disclosure is not limited to a particular limit of detection for the disclosed methods, although in certain aspects, the limit of detection for SFTVS is approximately one copy per sample.

Additional embodiments are described herein.

### BRIEF DESCRIPTION OF THE DRAWINGS

FIG. 1 shows development and performance evaluation of diagnostic tools for the detection of SFTSV. A) Schematic representation of the RT-qPCR assay developed for SFTSV detection, targeting two genomic segments (L and S) and both geographically divergent lineages (Lineage 1 and Lineage 2). B) Amplification curves obtained from 10-fold serial dilutions of specific in vitro transcripts (IVTs) from both genomic segments, representative of both lineages. C) Evaluation of the assay linear range and limit of detection (LOD) based on probit function analysis using the 10-fold dilution series.
FIG. 2 shows patient data.
FIG. 3 shows sequences of RT-qPCR primers and probes.
FIG. 4 shows mismatch analysis of RT-qPCR primers for detection of L segment.
FIG. 5 shows mismatch analysis of RT-qPCR primers for detection of S segment. A) set one primers. B) set two primers.

### DETAILED DESCRIPTION

Aspects of the present disclosure provide compositions, methods, and kits for detecting *Severe fever with thrombocytopenia syndrome virus* (SFTSV) nucleic acids.

The SFTSV virus has three segments (L, M, and S). Each segment has three Clades (Clade I, Clade II, and Clade III). Experiments described herein identified that the L segment is under purifying selection and the S segment is under positive selection, where new strains will emerge due to changes in S segment without affecting the L segment.

Accordingly, a detection assay that targeted the L segment to track the virus and multiple lineages (e.g., lineage 1 and 2) of the S segment for emergence of new strains was developed. This assay provides an improvement over existing assays that only target one segment or a subset of Clades, thus allowing mutational escape.

Section headings as used in this section and the entire disclosure herein are merely for organizational purposes and are not intended to be limiting.

### 1. Definitions

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present disclosure. All publications, patent applications, patents and other references mentioned herein are incorporated by reference in their entirety. The materials, methods, and examples disclosed herein are illustrative only and not intended to be limiting.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "an" and "the" include plural references unless the context clearly dictates otherwise. The present disclosure also contemplates other embodiments "comprising," "consisting of" and "consisting essentially of," the embodiments or elements presented herein, whether explicitly set forth or not.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

As used herein, the term "amplicon" refers to a nucleic acid generated via an amplification reaction. The amplicon is typically double stranded DNA; however, it may be RNA and/or a DNA:RNA hybrid. The amplicon comprises DNA complementary to a sample nucleic acid. In some embodiments, primer pairs are configured to generate amplicons from a sample nucleic acid. As such, the base composition of any given amplicon may include the primer pair (e.g., also referred to herein as a "primer set"), the complement of the primer pair, and the region of a sample nucleic acid that was amplified to generate the amplicon. In one embodiment, the incorporation of the designed primer pair sequences into an amplicon may replace the native sequences at the primer binding site, and complement thereof. In certain embodiments, after amplification of the target region using the primers, the resultant amplicons having the primer sequences are used for subsequent analysis (e.g. base composition determination, for example, via direct sequencing). In some embodiments, the amplicon further comprises a length that is compatible with subsequent analysis. An example of an amplicon is a DNA or an RNA product (usually a segment of a gene, DNA or RNA) produced as a result of PCR, real-time PCR, RT-PCR, competitive RT-PCR, ligase chain reaction (LCR), gap LCR, strand displacement amplification (SDA), nucleic acid sequence-based amplification (NASBA), transcription-mediated amplification (TMA), or the like.

As used herein, the phrases "amplifying", "amplification," "amplification method," or "amplification reaction," are used interchangeably herein, in the context of nucleic acids, refers to the production of multiple copies of a polynucleotide, or a portion of the polynucleotide, typically starting from a small amount of the polynucleotide (e.g., a single polynucleotide molecule), where the amplification products or amplicons are generally detectable. Amplification of polynucleotides encompasses a variety of chemical and enzymatic processes. Generation of multiple DNA copies from one or a few copies of a target or template DNA molecule during a polymerase chain reaction (PCR), isothermal reaction, or a ligase chain reaction (LCR) are forms of amplification. Amplification is not limited to the strict duplication of the starting molecule. For example, the generation of multiple cDNA molecules from a limited amount of RNA in a sample using reverse transcription (RT)-PCR is a form of amplification. Furthermore, the generation of multiple RNA molecules from a single DNA molecule during the process of transcription is also a form of amplification.

As used herein, the phrase "amplification conditions" refers to conditions that promote annealing and/or extension of one or more primer sequences (e.g., one or more primer sets). Such conditions are well-known in the art and depend on the amplification method selected. For example, PCR amplification conditions generally comprise thermal cycling, e.g., cycling of the reaction mixture between two or more temperatures. In isothermal amplification reactions, amplification occurs without thermal cycling although an initial temperature increase may be required to initiate the reaction. Amplification conditions encompass all reaction conditions including, but not limited to, temperature and temperature cycling, buffer, salt, ionic strength, pH, and the like.

As used herein, the phrase "amplification reagents" refers to reagents used in amplification reactions and may include, but is not limited to, buffers, reagents, enzymes having reverse transcriptase, and/or polymerase, or exonuclease activities; enzyme cofactors such as magnesium or manganese; salts; and deoxynucleotide triphosphates (dNTPs), such as deoxyadenosine triphosphate (dATP), deoxyguanosine triphosphate (dGTP), deoxycytidine triphosphate (dCTP), deoxythymidine triphosphate (dTTP), and deoxyuridine triphosphate (dUTP). Amplification reagents may readily be selected by one skilled in the art depending on the amplification method employed.

The terms "bead," "microparticle," and "particle" used herein interchangeably herein, refer to a substantially spherical solid support. The bead may be a magnetic bead or a magnetic particle. Microparticles that can be used herein can be any type known in the art. For example, the bead or microparticle can be a magnetic bead or magnetic particle. Magnetic beads/particles may be ferromagnetic, ferrimagnetic, paramagnetic, superparamagnetic or ferrofluidic. Exemplary ferromagnetic materials include Fe, Co, Ni, Gd, Dy, CrO₂, MnAs, MnBi, EuO, and NiO/Fe. Examples of ferrimagnetic materials include NiFe₂O₄, CoFe₂O4, Fe3O₄ (or FeO.Fe₂O₃). Beads can have a solid core portion that is magnetic and is surrounded by one or more non-magnetic layers. Alternately, the magnetic portion can be a layer around a non-magnetic core. The size of the microparticles used in the methods, compositions and kits described herein can vary. Preferably, the microparticles have a substantially uniform diameter of less than about 0.10 µm, less than about 0.20 µm, less than about 0.30 µm, less than about 0.40 µm, less than about 0.50 µm, less than about 0.60 µm, less than about 0.70 µm, less than about 0.80 µm, less than about 0.9 µm, less than about 1 µm, less than about 2 µm, less than about 3 µm, less than about 4 µm, or less than about 5 µm.

A "coding sequence" is a polynucleotide sequence which is transcribed into mRNA and translated into a polypeptide when placed under the control of appropriate regulatory sequences. The boundaries of the coding sequence are determined by and include a translation start codon at the 5'-terminus and one or more translation stop codons at the 3'-terminus. A coding sequence can include, but is not limited to, mRNA, cDNA, and recombinant polynucleotide sequences.

The terms "complement", "complementary" or "complementarity" are used herein in reference to "polynucleotides" and "oligonucleotides" (which are interchangeable terms that refer to a sequence of nucleotides) related by the base-pairing rules. These terms may also include mimics of or artificial bases that may not faithfully adhere to the base-pairing rules. For example, the sequence "C-A-G-T," is complementary to the sequence "G-T-C-A." Complementarity can be "partial" or "total." "Partial" complementarity is where one or more nucleic acid bases are not matched according to the base pairing rules. "Total" or "complete" complementarity between nucleic acids is where each and every nucleic acid base is matched with another base under the base pairing rules. The degree of complementarity between nucleic acid strands has significant effects on the efficiency and strength of hybridization between nucleic acid strands. This is of particular importance in amplification reactions, as well as detection methods which depend upon binding between nucleic acids.

The term "control sequence" refers to polynucleotide sequences which are necessary to affect the expression of coding sequences to which they are ligated. The nature of such control sequences differs depending upon the host organism. In prokaryotes, such control sequences generally include promoter, ribosomal binding site and terminators; in eukaryotes, such control sequences generally include promoters, terminators and, in some instances, enhancers. The term "control sequence" thus is intended to include at a minimum all components whose presence is necessary for expression, and also may include additional components whose presence is advantageous, for example, leader sequences.

As used herein, the phrase, "directly detectable," when used in reference to a detectable label or detectable moiety, means that the detectable label or detectable moiety does not require further reaction or manipulation to be detectable. For example, a fluorescent moiety is directly detectable by fluorescence spectroscopy methods. In contrast, the phrase "indirectly detectable," when used herein in reference to a detectable label or detectable moiety, means that the detectable label or detectable moiety becomes detectable after further reaction or manipulation. For example, a hapten becomes detectable after reaction with an appropriate antibody attached to a reporter, such as a fluorescent dye.

"Encoded by" refers to a nucleic acid sequence which codes for a polypeptide sequence. Also encompassed are polypeptide sequences which are immnunologically identifiable with a polypeptide encoded by the sequence. Thus, a "polypeptide," "protein," or "amino acid" sequence as claimed herein may have at least 60% similarity, more preferably at least about 70% similarity, and most preferably about 80% similarity to a particular polypeptide or amino acid sequence.

A "fragment" of a specified polypeptide refers to portions of an amino acid sequence. Fragments may range in size from 3 amino acid residues to the entire amino acid sequence minus one amino acid residue. In some aspects, the fragment is 3-5 amino acids in length, 8-10 amino acids in length, 10-15 amino acids in length, 10-20 amino acids in length, 10-25 amino acids in length, 10-30 amino acids in length, 10-40 amino acids in length, 10-50 amino acids in length, 10-60 amino acids in length, 10-70 amino acids in length, 10-80 amino acids in length, 10-90 amino acids in length, 10-100 amino acids in length, 10-150 amino acids in length, 10-200 amino acids in length, 10-300 amino acids in length, 10-400 amino acids in length, or 10-500 amino acids in length. A "fragment" of a specified nucleotide or polynucleotide sequence refers to portions of a nucleotide or polynucleotide sequence. Fragments may range in size from 5 nucleotide residues to the entire nucleotide sequence minus one nucleic acid residue. In some aspects, the fragment is 5-10 nucleic acids in length, 10-15 nucleic acids in length, 10-20 nucleic acids in length, 10-25 nucleic acids in length, 10-30 nucleic acids in length, 10-40 nucleic acids in length, 10-50 nucleic acids in length, 10-60 nucleic acids in length, 10-70 nucleic acids in length, 10-80 nucleic acids in length, 10-90 nucleic acids in length, 10-100 nucleic acids in length, 10-150 nucleic acids in length, 10-200 nucleic acids in length, 10-300 nucleic acids in length, 10-400 nucleic acids in length, or 10-500 nucleic acids in length.

As used herein, the term "gene" refers to deoxyribonucleotide or ribonucleotide sequences comprising the coding region of a structural gene and including sequences located adjacent to the coding region on both the 5' and 3' ends for a distance of about 1 kb on either end such that the gene corresponds to the length of the full-length mRNA. The sequences which are located 5' of the coding region and which are present on the mRNA are referred to as 5' non-translated sequences. The sequences which are located 3' or downstream of the coding region and which are present on the mRNA are referred to as 3' non-translated sequences. The term "gene" encompasses both amplified and genomic forms of a gene. A genomic form or clone of a gene contains the coding region interrupted with non-coding sequences termed "introns" or "intervening regions" or "intervening sequences." Introns are segments of a gene which are transcribed into heterogeneous nuclear RNA (hnRNA); introns may contain regulatory elements such as enhancers. Introns are removed or "spliced out" from the nuclear or primary transcript; introns therefore are absent in the messenger RNA (mRNA) transcript. The mRNA functions during translation to specify the sequence or order of amino acids in a nascent polypeptide.

As used herein, the term "genome" refers to the entirety of an organism's hereditary information that is encoded in its primary DNA or RNA or nucleotide sequence (DNA or RNA as applicable). The genome includes both the genes and the non-coding sequences. For example, the genome may represent a microbial genome, such as an archaeal genome, a fungal genome (e.g., yeast), a protozoan genome, or a viral genome.

As used herein, the term "hybridization" refers to the formation of complexes between nucleic acid sequences which are sufficiently complementary to form complexes via Watson-Crick base pairing or non-canonical base pairing. For example, when a primer "hybridizes" with a target sequence (template), such complexes (or hybrids) are sufficiently stable to serve the priming function required by, e.g., the DNA polymerase, to initiate DNA synthesis. It will be appreciated by one skilled in the art that hybridizing sequences need not have perfect complementarity to provide stable hybrids. In many situations, stable hybrids will form where fewer than about 10% of the bases are mismatches. Accordingly, as used herein, the term "complementary" refers to an oligonucleotide that forms a stable duplex with its complement under assay conditions, generally where there is about 80%, about 81%, about 82%, about 83%, about 84%, about 85%, about 86%, about 87%, about 88%, about 89%, about 90%, about 91%, about 92%, about 93%, about 94% about 95%, about 96%, about 97%, about 98%, or about 99% greater homology. Those skilled in the art understand how to estimate and adjust the stringency of hybridization conditions such that sequences having at least a desired level of complementarity will stably hybridize, while those having lower complementarity will not. Examples of hybridization conditions and parameters can be found, for example in, Sambrook et al., "Molecular Cloning: A Laboratory Manual" 1989, Second Edition, Cold Spring Harbor Press: Plainview, NY; F. M. Ausubel, "Current Protocols in Molecular Biology" 1994, John Wiley & Sons: Secaucus, NJ.

As used herein the phrase "hybridization product" refers to a complex formed between two nucleic acid sequences by virtue of the formation of hydrogen bounds between complementary G and C bases and between complementary A and T bases; these hydrogen bonds may be further stabilized by base stacking interactions. The two complementary nucleic acid sequences hydrogen bond in an antiparallel configuration. A hybridization product may be formed in solution or between one nucleic acid sequence present in solution and another nucleic acid sequence immobilized to a solid support (e.g., hybridization of a biotinylated probe used in target enrichment step (c) to a nucleic acid of interest and magnetic streptavidin beads).

As used herein, the terms "identification", "identify", and "identifying" refer to recognizing a specific microbe or microbes in a sample from a subject, for example, archaea, bacteria, fungi (e.g., yeast), protozoa, and/or viruses in sample from a subject.

As used herein, the term, "isolated" means that the referenced material, such as, for example, biological material (e.g., DNA, cDNA, RNA, mRNA, a nucleic acid, nucleotide, oligonucleotide, a protein, or a polypeptide) is free of components found in the natural environment in which the material is normally found. In particular, isolated biological material is free of cellular components. In the case of nucleic acid molecules, an isolated nucleic acid includes a PCR product, an isolated mRNA, a cDNA, an isolated genomic DNA, or a restriction fragment. In some embodiments, an isolated nucleic acid is preferably excised from the chromosome in which it may be found. Isolated nucleic acid molecules can be inserted into plasmids, cosmids, artificial chromosomes, and the like. Thus, in some embodiments, a recombinant nucleic acid is an isolated nucleic acid. An isolated protein may be associated with other proteins or nucleic acids, or both, with which it associates in the cell, or with cellular membranes if it is a membrane-associated protein. In some embodiments, an isolated material can be purified.

The terms "label", "detectable label" or "detectable moiety" are used interchangeably herein and refer to one or more atoms that can be specifically detected to indicate the presence of a substance to which the one or more atoms is attached. A label can be a primary label that is directly detectable or secondary label that can be indirectly detected, for example, via direct or indirect interaction with a primary. A label can be linked to primers, probes, or primers and probes either directly or indirectly. Labels include dyes, particles, chromophores (e.g., an atom or molecule that imparts a detectable color), combinatorial fluorescence energy transfer labels, electrophores, redox active moieties (e.g., transition metals), enzymes, haptens, luminescent compounds (e.g., bioluminescent, phosphorescent, or chemiluminescent moieties), fluorophores, fluorescent dyes, mass labels, and radiolabels. Labels and related detections methods are well known (See, e.g., U.S. Patent Nos. 5,201,015, 5,491,063, 5,591,578, 6,627,748; Styer and Haugland, Proc. Natl. Acad. Sci. U.S.A., 98:719 (1967)).

The term "fluorophore" refers a fluorescent chemical compound that can re-emit light upon light excitation. Fluorophores include, for example, fluorescent lanthanide complexes, including those of Europium and Terbium, fluorescein, rhodamine, tetramethylrhodamine, eosin, erythrosin, coumarin, methyl-coumarins, pyrene, Malacite green, Cy3, Cy5, CalFluor Red^{™}, CalFluor Orange^{™}, stilbene, Quasar dyes (e.g., Quasar 570, Quasar 670, Quasar 705), Lucifer Yellow, Cascade Blue^{™}, Texas Red, Alexa dyes, phycoerythin, Bodipy, and others known in the art, See, e.g., Haugland, Molecular Probes Handbook (Eugene, OR), 6th Edition; The Synthegen catalog (Houston, Tex.); Lakowicz, Principles of Fluorescence Spectroscopy, 2nd Ed., Plenum Press New York (1999), and WO 98/59066.

The term "quencher" as used herein refers to a molecule that absorbs light. Quenchers are commonly used in combination with a light emitting label such as a fluorophore to absorb emitted light when in close proximity to the fluorophore. Quenchers are well-known in the art and include, e.g., Black Hole Quencher^{™} (or BHQ^{™}, BHQ-1^{™}, or BHQ-2^{™}), Blackberry Quencher, Dabcyl, QSY, and Tamra^{™} compounds, to name a few.

A "homogeneous detectable label" as used herein refers to a label that associates with a probe oligonucleotide and that can be detected without physically removing hybridized from unhybridized forms of the label or labeled probe. Examples of homogeneous labels have been described in detail in, for example, U.S. Patent Nos. 5,283,174, 6,150,097, 5,201,015, 5,656,207, and 5,658,737.

Linear probes, molecular torches and beacons can be labeled with an interactive pair of detectable labels. Examples of detectable labels that are members of an interactive pair of detectable labels interact with each other by FRET or non-FRET energy transfer mechanisms. Fluorescence resonance energy transfer (FRET) involves the radiationless transmission of energy quanta from the site of absorption to the site of its utilization in the molecule, or system of molecules, by resonance interaction between chromophores, over distances considerably greater than interatomic distances, without conversion to thermal energy, and without the donor moiety and acceptor moiety coming into kinetic collision. The "donor" is the moiety that initially absorbs and then transfers the energy, and the "acceptor" is the moiety to which the energy is subsequently transferred. In addition to FRET, there are at least three other "non-FRET" energy transfer processes by which excitation energy can be transferred from a donor to an acceptor molecule.

When the two labels of a donor/acceptor pair are held sufficiently close that energy emitted by one label can be received or absorbed by the second label, whether by a FRET or non-FRET mechanism, the two labels are said to be in "energy transfer relationship" with each other. This is the case, for example, when a molecular beacon or molecular torch is maintained in the "closed" state by formation of a stem duplex, and fluorescent emission from a fluorophore attached to one arm of the probe is quenched by a quencher moiety on the opposite arm. This is also the case when, for example, a linear probe is labeled with a fluorophore and a quencher at a distance along the linear probe that fluorescent emission from the attached fluorophore is quenched by the attached quencher. In these instances, the spatial separation of the fluorophore and quencher molecules (e.g., by "opening" the molecular torch or beacon or by hydrolyzing the linear probe molecule).

Examples of donor/acceptor pairs, include fluorescein/tetramethylrhodamine, IAEDANS/fluorescein, EDANS/DABCYL, coumarin/DABCYL, fluorescein/fluorescein, BODIPY FL/BODIPY FL, fluorescein/DABCYL, lucifer yellow/DABCYL, BODIPY/DABCYL, eosine/DABCYL, erythrosine/DABCYL, tetramethylrhodamine/DABCYL, CalOrange/BHQ1, CalRed/BHQ2, FAM/BHQ1, Quasar/BHQ2, Texas Red/DABCYL, CY5/BH1, CY5/BH2, CY3/BH1, CY3/BH2 and fluorescein/QSY7 dye. Labels are available from LGC Biosearch Technologies (Petaluma, CA), Glen Research (Sterling, VA), Integrated DNA Technologies (Skokie, Il); Thermo Fisher (Waltham, MA), and others.

Synthetic techniques and methods of bonding labels to nucleic acids and detecting labels are well known in the art and can be used (e.g., see Sambrook, et al., Molecular Cloning, A Laboratory Manual, 2nd ed. (Cold Spring Harbor Laboratory Press, Cold Spring Harbor, N.Y., 1989), Chapter 10; U.S. Patent Nos. 5,658,737, 5,656,207, 5,547,842, 5,283,174, and 4,581,333, and published European Patent No. 0 747 706). A probe may optionally contain a fluorophore and a quencher. The nucleotide residues of the probe that combine with the target nucleic acid sequence need not be strictly contiguous, as may be the case with a detectable moiety internal to the sequence of the probe.

As used herein, "melting temperature" or "Tm" as used herein refers to the temperature at which a population of double-stranded nucleic acid molecules becomes half dissociated into single strands. As indicated by standard references, a simple estimate of the Tm value may be calculated by the equation: Tm=81.5+0.41 (% G+C), when a nucleic acid is in aqueous solution at 1M NaCl. Anderson et al, "Quantitative Filter Hybridization" In: Nucleic Acid Hybridization (1985). More sophisticated computations take structural, as well as sequence characteristics, into account for the calculation of Tm.

As used herein, the phrases "nucleic acid", "nucleotide", and "polynucleotide" and "nucleic acid sequence" and "nucleotide sequence" include a nucleic acid, an oligonucleotide, a nucleotide, a polynucleotide, and any fragment, variant, or derivative thereof. The nucleic acid or polynucleotide may be double- stranded, single- stranded, or triple-stranded DNA or RNA (including cDNA), or a DNA-RNA hybrid of genetic or synthetic origin, wherein the nucleic acid contains any combination of deoxyribonucleotides and ribonucleotides and any combination of bases, including, but not limited to, adenine, thymine, cytosine, guanine, uracil, inosine, and xanthine hypoxanthine. As further used herein, the term "cDNA" refers to an isolated DNA polynucleotide or nucleic acid molecule, or any fragment, derivative, or complement thereof. It may be double-stranded, single- stranded, or triple-stranded, it may have originated recombinantly or synthetically, and it may represent coding and/or noncoding 5' and/or 3' sequences.

As used herein, the phrases, "nucleic acid hybridization" or "hybridization" refer to anti-parallel hydrogen bonding between two single-stranded nucleic acids, in which A pairs with T (or U if an RNA nucleic acid) and C pairs with G. Nucleic acid molecules are "hybridizable" to each other when at least one strand of one nucleic acid molecule can form hydrogen bonds with the complementary bases of another nucleic acid molecule under defined stringency conditions. Stringency of hybridization is determined, e.g., by (i) the temperature at which hybridization and/or washing is performed, and (ii) the ionic strength and (iii) concentration of denaturants such as formamide of the hybridization and washing solutions, as well as other parameters. Hybridization requires that the two strands contain substantially complementary sequences. Depending on the stringency of hybridization, however, some degree of mismatches may be tolerated. Under "low stringency" conditions, a greater percentage of mismatches are tolerable (i.e., will not prevent formation of an anti-parallel hybrid).

As used herein, the term "oligonucleotide" refers to a nucleic acid, generally of at least 10, at least 15, at least 20 nucleotides, at least 30 nucleotides, at least 60 nucleotides, at least 80 nucleotides, at least 90 nucleotides, at least 100 nucleotides, at least 110 nucleotides, at least 120 nucleotides, at least 130 nucleotides, at least 140 nucleotides, at least 150 nucleotides, at least 160 nucleotides, or at least 180 nucleotides, that is hybridizable to a genomic DNA molecule, a cDNA molecule, or an mRNA molecule encoding a gene, mRNA, cDNA, or other nucleic acid of interest. The nucleic acids that comprise the oligonucleotides include but are not limited to DNA, RNA, linked nucleic acids (LNA), bridged nucleic acids (BNA) and peptide nucleic acids. (PNA). Oligonucleotides can be labeled, e.g., with 32 P-nucleotides or nucleotides to which a label, such as biotin, has been covalently conjugated.

"Operably linked" refers to a situation wherein the components described are in a relationship permitting them to function in their intended manner. Thus, for example, a control sequence "operably linked" to a coding sequence is ligated in such a manner that expression of the coding sequence is achieved under conditions compatible with the control sequences.

As used herein, the phrases "percent (%) sequence identity", or "sequence identity" as used interchangeably herein refer to the degree of identity or correspondence between different nucleotide sequences of nucleic acid molecules (e.g., oligonucleotides, polynucleotides, etc.) or amino acid sequences of proteins that may or may not share a common evolutionary origin. Sequence identity can be determined using any publicly available sequence comparison algorithms, such as, for example, BLAST, FASTA, DNA Strider, and GCG (Genetics Computer Group, Program Manual for the GCG Package, Version 7, Madison, Wisconsin).

To determine the percent identity between two amino acid sequences or two nucleic acid molecules, the sequences are aligned for optimal comparison purposes. The percent identity between the two sequences is a function of the number of identical positions shared by the sequences (i.e., percent identity = number of identical positions/total number of positions (e.g., overlapping positions) x 100). In one embodiment, the two sequences are, or are about, of the same length. The percent identity between two sequences can be determined using techniques similar to those described below, with or without allowing gaps. In calculating percent sequence identity, typically exact matches are counted.

As used herein, the phrase "polymerase chain reaction" ("PCR") refers to the method disclosed in U.S. Patent Nos. 4,683,195 and 4,683,202, herein incorporated by reference, which describe a method for increasing the concentration of a segment of a target sequence in a mixture of genomic DNA without cloning or purification. The length of the amplified segment of the desired target sequence is determined by the relative positions of two oligonucleotide primers with respect to each other, and therefore, this length is a controllable parameter. By virtue of the repeating aspect of the process, the method is referred to as the "polymerase chain reaction" (hereinafter "PCR"). Because the desired amplified segments of the target sequence become the predominant sequences (in terms of concentration) in the mixture, they are said to be "PCR amplified". With PCR, it is possible to amplify a single copy of a specific target sequence in genomic DNA to a level detectable by several different methodologies (e.g., hybridization with a labeled probe; incorporation of biotinylated primers followed by avidin-enzyme conjugate detection; incorporation of 32P-labeled deoxynucleotide triphosphates, such as dCTP or dATP, into the amplified segment). In addition to genomic DNA, any oligonucleotide sequence can be amplified with the appropriate set of primer molecules. In particular, the amplified segments created by the PCR process itself are, themselves, efficient templates for subsequent PCR amplification. With PCR, it is also possible to amplify a complex mixture (library) of linear DNA molecules, provided they carry suitable universal sequences on either end such that universal PCR primers bind outside of the DNA molecules that are to be amplified.

Any suitable PCR methodology, combination of PCR methodologies, or combination of amplification techniques may be utilized in the methods (e.g., droplet-based detection, separation, and/or sequencing of target nucleic acids) disclosed herein, such as cdPCR, dPCR LATE, allele-specific PCR, assembly PCR, asymmetric PCR, digital PCR, endpoint PCR, hot-start PCR, *in situ* PCR, intersequence-specific PCR, inverse PCR, linear after exponential PCR, ligation-mediated PCR, methylation-specific PCR, miniprimer PCR, multiplex ligation-dependent probe amplification, multiplex PCR, nested PCR, overlap-extension PCR, polymerase cycling assembly, qualitative PCR, quantitative PCR, real-time PCR, RT-PCR, single-cell PCR, solid-phase PCR, thermal asymmetric interlaced PCR, touchdown PCR, or universal fast walking PCR, etc.

"Polypeptide" and "protein" are used interchangeably herein and indicate a molecular chain of amino acids linked through covalent and/or noncovalent bonds. The terms do not refer to a specific length of the product. Thus, peptides, oligopeptides and proteins are included within the definition of polypeptide. The terms include post-expression modifications of the polypeptide, for example, glycosylations, acetylations, phosphorylations and the like. In addition, protein fragments, analogs, mutated or variant proteins, fusion proteins and the like are included within the meaning of polypeptide.

The term "primer" or "oligonucleotide primer" as used interchangeably herein as used herein, refers to an oligonucleotide capable of acting as a point of initiation for DNA synthesis under suitable conditions. Suitable conditions include those in which hybridization of the oligonucleotide to a template nucleic acid occurs, and synthesis or amplification of the target sequence occurs, in the presence of four different nucleoside triphosphates and an agent for extension (e.g., a DNA polymerase) in an appropriate buffer and at a suitable temperature. A "forward oligonucleotide primer", "forward primer", or "sense primer," as used interchangeably herein, refers to an oligonucleotide capable of acting as a point of initiation for DNA synthesis at the 5' end of a target nucleic acid sequence. A "reverse oligonucleotide primer", "reverse primer", or "antisense primer," as used interchangeably herein, refers to an oligonucleotide capable of acting as a point of initiation for DNA synthesis at the 3' end of a target nucleic acid sequence. The phrase "forward primer" refers to a primer that hybridizes (or anneals) with the target sequence (e.g., template strand). The phrase "reverse primer" refers to a primer that hybridizes (or anneals) to the complementary strand of the target sequence. The forward primer hybridizes with the target sequence 5' with respect to the reverse primer. The phrase "forward primer" refers to a primer that hybridizes (or anneals) with the target sequence (e.g., template strand). The phrase "reverse primer" refers to a primer that hybridizes (or anneals) to the complementary strand of the target sequence. The forward primer hybridizes with the target sequence 5' with respect to the reverse primer.

As used herein, the phrase "primer set" refers to two or more primers which together are capable of priming the amplification of a target sequence or target nucleic acid of interest (e.g., a target sequence within SFTSV). In certain embodiments, the term "primer set" refers to a pair of primers including a 5' (upstream) primer (or forward primer) that hybridizes with the 5 '-end of the target sequence or target nucleic acid to be amplified and a 3' (downstream) primer (or reverse primer) that hybridizes with the complement of the target sequence or target nucleic acid to be amplified. In certain embodiments, a primer set includes at least one forward primer and at least one reverse primer. Such primer sets or primer pairs are particularly useful in PCR amplification reactions.

The term "probe" or "oligonucleotide probe" as used interchangeably herein, refers to an oligonucleotide that hybridizes specifically to a target sequence in a nucleic acid, preferably in an amplified nucleic acid, under conditions that promote hybridization, to form a detectable hybrid. A probe may contain a detectable moiety (e.g., a label) which either may be attached to the end(s) of the probe or may be internal. The nucleotides of the probe which hybridize to the target nucleic acid sequence need not be strictly contiguous, as may be the case with a detectable moiety internal to the sequence of the probe. Detection may either be direct (i.e., resulting from a probe hybridizing directly to the target sequence or amplified nucleic acid) or indirect (i.e., resulting from a probe hybridizing to an intermediate molecular structure that links the probe to the target sequence or amplified nucleic acid). An oligonucleotide probe may comprise target-specific sequences and other sequences that contribute to three-dimensional conformation of the probe (e.g., as described in, e.g., U.S. Patent Nos. 5,118,801 and 5,312,728).

"Purified polypeptide" or "purified polynucleotide" refers to a polypeptide or polynucleotide of interest or fragment thereof which contains less than about 50%, less than about 70%, less than about 71%, less than about 72%, less than about 73%, less than about 74%, less than about 75%, less than about 76%, less than about 77%, less than about 78%, less than about 79%, less than about 80%, less than about 81%, less than about 82%, less than about 83%, less than about 84%, less than about 85%, less than about 86%, less than about 87%, less than about 88%, less than about 89%, or less about 90% of cellular components with which the polypeptide or polynucleotide of interest or fragment thereof is naturally associated. Methods for purifying are known in the art.

The terms "recombinant polypeptide" or "recombinant protein", used interchangeably herein, describe a polypeptide which by virtue of its origin or manipulation is not associated with all or a portion of the polypeptide with which it is associated in nature and/or is linked to a polypeptide other than that to which it is linked in nature. A recombinant or encoded polypeptide or protein is not necessarily translated from a designated nucleic acid sequence. It also may be generated in any manner, including chemical synthesis or expression of a recombinant expression system.

"Recombinant host cells," "host cells," "cells," "cell lines," "cell cultures," and other such terms denoting microorganisms or higher eukaryotic cell lines cultured as unicellular entities refer to cells which can be, or have been, used as recipients for recombinant vector or other transferred DNA, and include the original progeny of the original cell which has been transfected.

As used herein "replicon" means any genetic element, such as a plasmid, a chromosome or a virus, that behaves as an autonomous unit of polynucleotide replication within a cell.

As used herein, the term "sample", "biological sample" or "test sample", as all used interchangeably herein generally refers to a biological material being tested for and/or suspected of containing an analyte of interest, such as SFTSV. In some aspects, the sample is suspected of containing an SFTSV nucleic acid. The sample may be derived from any biological source, such as, a cervical, vaginal or anal swab or brush, or a physiological fluid including, but not limited to, whole blood, serum, plasma, interstitial fluid, saliva, ocular lens fluid, cerebral spinal fluid, sweat, urine, milk, ascites fluid, mucus, nasal fluid, sputum, synovial fluid, peritoneal fluid, vaginal fluid, menses, amniotic fluid, semen, and so forth. The sample may be used directly as obtained from the biological source or following a pretreatment to modify the character of the sample. For example, such pretreatment may include preparing plasma from blood, diluting viscous fluids, and so forth. Methods of pretreatment may also involve filtration, precipitation, dilution, distillation, mixing, concentration, lyophilization, inactivation of interfering components, the addition of reagents, lysing, etc. Moreover, it may also be beneficial to modify a solid sample to form a liquid medium or to release the analyte. Preferably, the sample may be plasma.

As used herein, the term "sequencing" refers to any methods for determining the order of the nucleotide bases, adenine, guanine, cytosine, and thymine, in a molecule of DNA.

As used herein, the phrase "statistically significant" refers to the likelihood that a relationship between two or more variables is caused by something other than random chance. Statistical hypothesis testing is used to determine whether the result of a data set is statistically significant. In statistical hypothesis testing, a statistically significant result is attained whenever the observed p-value of a test statistic is less than the significance level defined of study. The p-value is the probability of obtaining results at least as extreme as those observed, given that the null hypothesis is true. Examples of statistical hypothesis analysis include Wilcoxon signed-rank test, t-test, Chi-Square or Fisher's exact test. As used herein, the term "significant" refers to a change that has not been determined to be statistically significant (e.g., it may not have been subject to statistical hypothesis testing).

As used herein, the term "stringency" is used in reference to the conditions of temperature, ionic strength, and the presence of other compounds such as organic solvents, under which nucleic acid hybridizations are conducted. "Stringency" typically occurs in a range from about Tm to about 20°C to 25°C below Tm. A "stringent hybridization" can be used to identify or detect identical polynucleotide sequences or to identify or detect similar or related polynucleotide sequences. For example, when fragments are employed in hybridization reactions under stringent conditions the hybridization of fragments which contain unique sequences (i.e., regions which are either non-homologous to or which contain less than about 50% homology or complementarity) are favored. Alternatively, when conditions of "weak" or "low" stringency are used hybridization may occur with nucleic acids that are derived from organisms that are genetically diverse (i.e., for example, the frequency of complementary sequences is usually low between such organisms).

The terms "subject" and "patient" as used herein interchangeably refers to any vertebrate, including, but not limited to, a mammal (e.g., cow, pig, camel, llama, horse, goat, rabbit, sheep, hamsters, guinea pig, cat, dog, rat, and mouse, a non-human primate (for example, a monkey, such as a cynomolgus or rhesus monkey, chimpanzee, etc.) and a human). In some embodiments, the subject may be a human or a non-human. In some embodiments, the subject is a human. The subject or patient may be undergoing other forms of treatment. The subject or patient may be undergoing monitoring of the course of a bacterial infection and/or treatment of a bacterial infection. The subject or patient may have or be suspected of having a viral infection and/or a symptom, disease, condition, or disorder associated with the bacterial infection. In some aspects, the subject or patient may have or be suspected of having a SFTSV infection. In other aspects, the subject or patient may have or be suspected of having a SFTSV infection. In yet other aspects, the subject or patient may have or be suspected of having a SFTSV infection, and have one or more systems of SFTSV infection.

"Statistically significant" as used herein refers to the likelihood that a relationship between two or more variables is caused by something other than random chance. Statistical hypothesis testing is used to determine whether the result of a data set is statistically significant. In statistical hypothesis testing, a statistically significant result is attained whenever the observed p-value of a test statistic is less than the significance level defined of the study. The p-value is the probability of obtaining results at least as extreme as those observed, given that the null hypothesis is true. Examples of statistical hypothesis analysis include Wilcoxon signed-rank test, t-test, Chi-Square or Fisher's exact test. "Significant" as used herein refers to a change that has not been determined to be statistically significant (e.g., it may not have been subject to statistical hypothesis testing).

The phrases "target sequence" and "target nucleic acid" are used interchangeably herein and refer to that which the presence or absence of which is desired to be detected. In the context of the present disclosure, a target sequence preferably includes a nucleic acid sequence to which one or more primers will complex. The target sequence can also include a probe-hybridizing region with which a probe will form a stable hybrid under appropriate amplification conditions. As will be recognized by one of ordinary skill in the art, a target sequence may be single-stranded or double-stranded.

"Treat," "treating" or "treatment" are each used interchangeably herein to describe reversing, alleviating, or inhibiting the progress of a disease and/or injury, or one or more symptoms of such disease, to which such term applies. Depending on the condition of the subject, the term also refers to preventing a disease, and includes preventing the onset of a disease, or preventing the symptoms associated with a disease. Treatment may be either performed in an acute or chronic way. The term also refers to reducing the severity of a disease or symptoms associated with such disease prior to affliction with the disease. Such prevention or reduction of the severity of a disease prior to affliction refers to administration of a pharmaceutical composition to a subject that is not at the time of administration afflicted with the disease. "Preventing" also refers to preventing the recurrence of a disease or of one or more symptoms associated with such disease. "Treatment" and "therapeutically," refer to the act of treating, as "treating" is defined above.

A "vector" is a replicon to which another polynucleotide segment is attached, such as to bring about the replication and/or expression of the attached segment.

Unless otherwise defined herein, scientific and technical terms used in connection with the present disclosure shall have the meanings that are commonly understood by those of ordinary skill in the art. For example, any nomenclatures used in connection with, and techniques of, cell and tissue culture, molecular biology, immunology, microbiology, genetics and protein and nucleic acid chemistry and hybridization described herein are those that are well known and commonly used in the art. The meaning and scope of the terms should be clear; in the event, however of any latent ambiguity, definitions provided herein take precedent over any dictionary or extrinsic definition. Further, unless otherwise required by context, singular terms shall include pluralities and plural terms shall include the singular.

### 2. SFTSV primers and probes

In some aspects, provided herein are compositions and methods for detecting SFTSV in a sample from a subject.

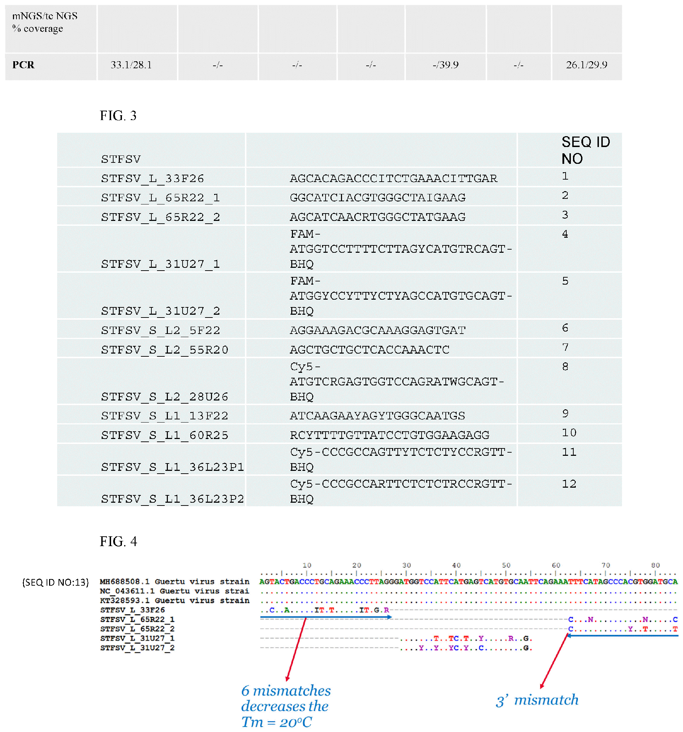

For example, in some aspects, provided herein are primers, probes, primer sets, primer sets and probes, compositions and kits for detecting human SFTSV nucleic acids in a subject. Exemplary primer and/or probe sets are described herein and shown in FIG. 3.

In some embodiments, provided herein are primers for amplifying SFTSV in a sample. In some embodiments, the primer is any suitable primer derived from SEQ ID NO: 1, SEQ ID NO: 2, SEQ ID NO: 3, SEQ ID NO: 6, SEQ ID NO: 7, SEQ ID NO: 9, or SEQ ID NO: 10 or fragments thereof. In some embodiments, the primer is any suitable primer that is a complement derived from SEQ ID NO: 1, SEQ ID NO: 2, SEQ ID NO: 3, SEQ ID NO: 6, SEQ ID NO: 7, SEQ ID NO: 9, or SEQ ID NO: 10, or fragments thereof.

In some embodiments, the primer has 80% or more sequence identity (e.g., 81%, 82%, 83%, 84%, 85%, 86%, 87%, 88%, 89%, 90%, 91%, 92%, 93%, 94%, 95%, 96%, 97%, 98%, 99%, or 100%) to the sequence of SEQ ID NO: 1, SEQ ID NO: 2, SEQ ID NO: 3, SEQ ID NO: 6, SEQ ID NO: 7, SEQ ID NO: 9, or SEQ ID NO: 10, or complements thereof.

In some embodiments, the primer is labeled with a detectable label. One or more primers (e.g., the one or more primers can be: (i) derived from SEQ ID NO: 1, SEQ ID NO: 2, SEQ ID NO: 3, SEQ ID NO: 6, SEQ ID NO: 7, SEQ ID NO: 9, or SEQ ID NO: 10, or fragments thereof; or (ii) a complement derived from SEQ ID NO: 1, SEQ ID NO: 2, SEQ ID NO: 3, SEQ ID NO: 6, SEQ ID NO: 7, SEQ ID NO: 9, or SEQ ID NO: 10, or fragments thereof), may be labeled with a detectable label.

In some aspects, provided herein are probes for detecting SFTSV. In some embodiments, the probe is any suitable probe derived from SEQ ID NO: 4, SEQ ID NO: 5, SEQ ID NO: 8, SEQ ID NO: 11, SEQ ID NO: 12 or fragments thereof. In some embodiments, the probe is a complement derived from SEQ ID NO: 4, SEQ ID NO: 5, SEQ ID NO: 8, SEQ ID NO: 11, SEQ ID NO: 12, or fragments thereof. In some embodiments, provided herein is a probe for detecting SFTSV in a sample, wherein the probe has a sequence having 80% or more sequence identity to a sequence of SEQ ID NO: 4, SEQ ID NO: 5, SEQ ID NO: 8, SEQ ID NO: 11, SEQ ID NO: 12, or complements thereof. For example, the probe may have 80%, 81%, 82%, 83%, 84% 85%, 86%, 87%, 88%, 89%, 90%, 91%, 92%, 93%, 94%, 95%, 96%, 97%, 98%, 99%, or 100% sequence identity to SEQ ID NO: 4, SEQ ID NO: 5, SEQ ID NO: 8, SEQ ID NO: 11, SEQ ID NO: 12, or complements thereof.

In some embodiments, the probe is labeled with a detectable label. In some aspects, one or more probes can be: (i) derived from SEQ ID NO: 4, SEQ ID NO: 5, SEQ ID NO: 8, SEQ ID NO: 11, SEQ ID NO: 12, or fragments thereof; or (ii) a complement derived from SEQ ID NO: 4, SEQ ID NO: 5, SEQ ID NO: 8, SEQ ID NO: 11, SEQ ID NO: 12, or fragments thereof, are labeled with a detectable label.

In some aspects, provided herein are one or more primers described in FIG. 6 or a complement thereof.

As shown in FIG. 3, forward primers of SEQ ID NO: 1 or a complement thereof and reverse primers of SEQ ID NO: 2 and/or SEQ ID NO: 3 can be used to detect the L segment of SFTSV.

The forward primer of SEQ ID NO: 6 or SEQ ID NO: 9 or a complement thereof and the reverse primer of SEQ ID NO: 7 or SEQ ID NO: 10 or a complement thereof, can be used to detect the S segment of SFTSV.

In other aspects, provided herein is a primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1, SEQ ID NO: 2, SEQ ID NO: 3, SEQ ID NO: 6, SEQ ID NO: 7, SEQ ID NO: 9, or SEQ ID NO: 10, or a complement thereof.

In some aspects, provided herein are compositions for amplifying SFTSV S and/or L segments in a sample. The composition may comprise two or more primers as disclosed herein (e.g., a primer set, such a forward primer and a reverse primer). The compositions described herein may contain one (e.g., a single) primer set (comprising a forward primer and a reverse primer), two primer sets (comprising a first forward primer and a first reverse primer, which together comprise a first primer set, and a second forward primer and a second reverse primer, which together comprise a second primer set), three primer sets (comprising a first forward primer and a first reverse primer, which together comprise a first primer set, a second forward primer and a second reverse primer, which together comprise a second primer set, and a third forward primer and a third reverse primer, which together comprise a third primer set), four primer sets (comprising a first forward primer and a first reverse primer, which together comprise a first primer set, a second forward primer and a second reverse primer, which together comprise a second primer set, a third forward primer and a third reverse primer, which together comprise a third primer set, and a fourth forward primer and a fourth reverse primer, which together comprise a fourth primer set), or five primer sets (comprising a first forward primer and a first reverse primer, which together comprise a first primer set, a second forward primer and a second reverse primer, which together comprise a second primer set, a third forward primer and a third reverse primer, which together comprise a third primer set, a fourth forward primer and a fourth reverse primer, which together comprise a fourth primer set, and a fifth forward primer and a fifth reverse primer, which together comprise a fifth primer set).

In some embodiments, the composition comprises at least one forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to the sequence of SEQ ID NO: 1, SEQ ID NO: 6, SEQ ID NO: 9 or a complement thereof, and at least one reverse primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2, SEQ ID NO: 3, SEQ ID NO: 7, SEQ ID NO: 10, or a complement thereof.

In some embodiments, the composition comprises at least one forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof and at least one reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 or a complement thereof. In the composition, the forward primer of SEQ ID NO:1 or a complement thereof and the reverse primer of SEQ ID NO: 2 or a complement thereof can be used to detect an L segment of SFTSV.

In some embodiments, the composition comprises at least one forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof and at least one reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 3 or a complement thereof. In the composition, the forward primer of SEQ ID NO:1 or a complement thereof and the reverse primer of SEQ ID NO: 3 or a complement thereof can be used to detect an L segment of SFTSV.

In some embodiments, the composition comprises at least one forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 6 or a complement thereof and at least one reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 7 or a complement thereof. In the composition, the forward primer of SEQ ID NO: 6 or a complement thereof and the reverse primer of SEQ ID NO: 7 or a complement thereof can be used to detect an S segment of SFTSV.

In some embodiments, the composition comprises at least one forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 9 or a complement thereof and at least one reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 10 or a complement thereof. In the composition, the forward primer of SEQ ID NO: 9 or a complement thereof and the reverse primer of SEQ ID NO: 10 or a complement thereof can be used to detect an S segment of SFTSV.

In some embodiments, the composition comprises at least two sets of primers, such as a first set of primers and a second set of primers. In one aspect, the first set of primers comprises at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof and at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof. The second set of primers comprises at least one second forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 6 or a complement thereof and at least one second reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 7 or a complement thereof. In the composition, the forward primers of SEQ ID NO: 1, SEQ ID NO: 6 or a complement thereof and the reverse primers of SEQ ID NO: 2, SEQ ID NO: 3, SEQ ID NO: 7 or a complement thereof can be used to detect L and S segments of SFTSV.

In some embodiments, the composition comprises at least two sets of primers, such as a first set of primers and a second set of primers. In one aspect, the first set of primers comprises at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof and at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof. The second set of primers comprises at least one second forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 9 or a complement thereof and at least one second reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 10 or a complement thereof. In the composition, the forward primers of SEQ ID NO: 1, SEQ ID NO: 9 or a complement thereof and the reverse primers of SEQ ID NO: 2, SEQ ID NO: 3, SEQ ID NO: 10 or a complement thereof can be used to detect L and S segments of SFTSV.

In some embodiments, the composition comprises at least three sets of primers, such as a first set of primers, a second set of primers, and a third set of primers. In one aspect, the first set of primers comprises at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof and at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof. The second set of primers comprises at least one second forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 6 or a complement thereof and at least one second reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 7 or a complement thereof. The third set of primers comprises at least one third forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 9 or a complement thereof and at least one third reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 10 or a complement thereof. In the composition, the forward primers of SEQ ID NO: 1, SEQ ID NO: 6, SEQ ID NO: 9 or a complement thereof and the reverse primers of SEQ ID NO: 2, SEQ ID NO: 3, SEQ ID NO: 7, SEQ ID NO: 10 or a complement thereof can be used to detect L and S segments of SFTSV.

In some embodiments, the composition further comprises at least one probe. The composition may further comprise any probe described herein. In some aspects, provided herein are one or more probes described in FIG. 3.

As shown in FIG. 6, probes of SEQ ID NO: 4, SEQ ID NO: 5, or a complement thereof detect an L segment of SFTSV. Probes of SEQ ID NO: 8, SEQ ID NO; 11, SEQ ID NO: 12 or a complement thereof, can be used to detect an S segment of SFTSV.

In other aspects, provided herein is a probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 4, SEQ ID NO: 5, SEQ ID NO: 8, SEQ ID NO:11, SEQ ID NO: 12, or a complement thereof.

In some aspects, provided herein are compositions for amplifying SFTSV S and L segments in a sample. The composition may comprise two or more primers and a probe as disclosed herein (e.g., a primer set, such one or more forward primers and one or more reverse primers as well as one or more probes). While each primer and probe set may generate a single amplicon, in some embodiments, each primer and probe set may comprises one or more forward primers, reverse primers, and probes.

The compositions described herein may contain one (e.g., a single) primer set and one or more probes (comprising one or more forward primers, one or more reverse primers, and one or more probes), two primer sets and at least two probes (comprising a first forward primer(s) and a first reverse primer(s), which together comprise a first primer set and a first probe(s), and a second forward primer(s) and a second reverse primer(s), which together comprise a second primer set, and a second probe(s)), three primer sets and at least three probes (comprising a first forward primer(s) and a first reverse primer(s), which together comprise a first primer set and a first probe(s), a second forward primer(s) and a second reverse primer(s), which together comprise a second primer set, and a second probe(s), and a third forward primer(s) and a third reverse primer(s), which together comprise a third primer set, and a third probe(s)), four primer sets and at least four probes (comprising a first forward primer(s) and a first reverse primer(s), which together comprise a first primer set, and a first probe(s), a second forward primer(s) and a second reverse primer(s), which together comprise a second primer set, and a second probe(s), a third forward primer(s) and a third reverse primer(s), which together comprise a third primer set, and a third probe(s), and a fourth forward primer(s) and a fourth reverse primer(s), which together comprise a fourth primer set, and a fourth probe(s)), or five primer sets and at least five probes (comprising a first forward primer(s) and a first reverse primer(s), which together comprise a first primer set, and a first probe(s), a second forward primer(s) and a second reverse primer(s), which together comprise a second primer set, and a second probe(s), a third forward primer(s) and a third reverse primer(s), which together comprise a third primer set, and a third probe(s), a fourth forward primer(s) and a fourth reverse primer(s), which together comprise a fourth primer set, and fourth probe(s), and a fifth forward primer(s) and a fifth reverse primer(s), which together comprise a fifth primer set and a fifth probe(s)).

In some embodiments, the composition comprises at least one forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to the sequence of SEQ ID NO: 1, SEQ ID NO: 6, SEQ ID NO: 9 or a complement thereof, and at least one reverse primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2, SEQ ID NO: 3, SEQ ID NO: 7, SEQ ID NO: 10, or a complement thereof, and at least one probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 4, SEQ ID NO: 5, SEQ ID NO: 8, SEQ ID NO: 11, SEQ ID NO: 12, or a complement thereof.

In some embodiments, the composition comprises at least one forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof, at least one reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof, and at least one probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 4 and/or 5, or a complement thereof. In the composition, the forward primer of SEQ ID NO: 1 or a complement thereof, the reverse primer of SEQ ID NO: 2 and/or 3 or a complement thereof, and the probe of SEQ ID NO: 4 and/or 5 or a complement thereof can be used to detect an L segment of SFTSV.

In some embodiments, the composition comprises at least one forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 6 or a complement thereof, at least one reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 7 or a complement thereof, and at least one probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 8, or a complement thereof. In the composition, the forward primer of SEQ ID NO: 6 or a complement thereof, the reverse primer of SEQ ID NO: 7 or a complement thereof, and the probe of SEQ ID NO: 8 or a complement thereof can be used to detect an S segment of SFTSV.

In some embodiments, the composition comprises at least one forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 9 or a complement thereof, at least one reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 10 or a complement thereof, and at least one probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 11 and/or 12, or a complement thereof. In the composition, the forward primer of SEQ ID NO: 9 or a complement thereof, the reverse primer of SEQ ID NO: 10 or a complement thereof, and the probe of SEQ ID NO: 11 and/or 12 or a complement thereof can be used to detect an S segment of SFTSV.

In some embodiments, the composition comprises at least two sets of primers and at least two probes, such as a first set of primers and a first probe(s) and a second set of primers and a second probe(s). In one aspect, the first set of primers comprises at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof, at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof, and at least one first probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 4 and/or 5, or a complement thereof. The second set of primers comprises at least one second forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 6 or a complement thereof, at least one second reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 7 or a complement thereof, and at least one second probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 8, or a complement thereof. In the composition, the forward primers of SEQ ID NO: 1, SEQ ID NO: 6 or a complement thereof, the reverse primers of SEQ ID NO: 2, SEQ ID NO: 3, SEQ ID NO: 7 or a complement thereof, and the probes of SEQ ID NO: 4, SEQ ID NO: 5, SEQ ID NO: 8 or a complement thereof, can be used to detect the L and S segments of SFTSV.

In some embodiments, the composition comprises at least two sets of primers and at least two probes, such as a first set of primers and a first probe(s) and a second set of primers and a second probe(s). In one aspect, the first set of primers comprises at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof, at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof, and at least one first probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 4 and/or 5, or a complement thereof. The second set of primers comprises at least one second forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 9 or a complement thereof, at least one second reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 10 or a complement thereof, and at least one second probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 11 and/or 12, or a complement thereof. In the composition, the forward primers of SEQ ID NO: 1, SEQ ID NO: 9 or a complement thereof, the reverse primers of SEQ ID NO: 2, SEQ ID NO: 3, SEQ ID NO: 10 or a complement thereof, and the probes of SEQ ID NO: 4, SEQ ID NO: 5, SEQ ID NO: 11, SEQ ID NO: 12, or a complement thereof, can be used to detect the L and S segments of SFTSV.

In some embodiments, the composition comprises at least three sets of primers, such as a first set of primers, a second set of primers, and a third set of primers. In one aspect, the first set of primers comprises at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof, at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof, and at least one first probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 4 and/or 5, or a complement thereof. The second set of primers comprises at least one second forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 6 or a complement thereof, at least one second reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 7 or a complement thereof, and at least one second probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 8, or a complement thereof. The third set of primers comprises at least one third forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 9 or a complement thereof, at least one third reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 10 or a complement thereof, and at least one third probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 11 and/or 12, or a complement thereof. In the composition, the forward primers of SEQ ID NO: 1, SEQ ID NO: 6, SEQ ID NO: 9 or a complement thereof, the reverse primers of SEQ ID NO: 2, SEQ ID NO: 3, SEQ ID NO: 7, SEQ ID NO: 9 or a complement thereof, and the probes of SEQ ID NO: 4, SEQ ID NO: 5 SEQ ID NO: 8, SEQ ID NO: 11, SEQ ID NO: 12 or a complement thereof, can be used to detect the L and S segments of SFTSV.

One or more oligonucleotide analogues can be prepared based on the primers and probes of the present disclosure. Such analogues may contain alternative structures such as peptide nucleic acids or "PNAs" (e.g., molecules with a peptide-like backbone instead of the phosphate sugar backbone of naturally occurring nucleic acids) and the like. These alternative structures are also encompassed by the primers and probes of the present disclosure. Similarly, it is understood that the primers and probes of the present disclosure may contain deletions, additions and/or substitutions of nucleic acid bases, to the extent that such alterations do not negatively affect the properties of these sequences. In particular, the alterations should not result in a significant decrease of the hybridizing properties of the primers and probes described herein. The primers and probes of the present disclosure may be prepared by any of a variety of methods known in the art (See, for example, Sambrook et al., "Molecular Cloning. A Laboratory Manual," 1989, 2. Supp. Ed., Cold Spring Harbour Laboratory Press: New York, NY; "PCR Protocols. A Guide to Methods and Applications ," 1990, M. A. Innis (Ed.), Academic Press: New York, NY; P. Tijssen "Hybridization with Nucleic Acid Probes- Laboratory Techniques in Biochemistry and Molecular Biology (Parts I and II)," 1993, Elsevier Science; "PCR Strategies," 1995, M. A. Innis (Ed.), Academic Press: New York, NY; and *"*Short Protocols in Molecular Biology", 2002, F. M. Ausubel (Ed.), 5. Supp. Ed., John Wiley & Sons: Secaucus, NJ). For example, primers and probes described herein may be prepared by chemical synthesis and polymerization based on a template as described, for example, in Narang et al., Meth. Enzymol., 1979, 68: 90-98; Brown et al., Meth. Enzymol., 1979, 68: 109-151 and Belousov et al., Nucleic Acids Res., 1997, 25: 3440-3444).

Syntheses may be performed on oligo synthesizers, such as those commercially available from Perkin Elmer/Applied Biosystems, Inc. (Foster City, CA), DuPont (Wilmington, DE) or Milligen (Bedford, MA). Alternatively, the primers and probes of the present disclosure may be custom made and ordered from a variety of commercial sources well-known in the art, including, for example, the Midland Certified Reagent Company (Midland, TX), ExpressGen, Inc. (Chicago, IL), Operon Technologies, Inc. (Huntsville, AL), BioSearch Technologies, Inc. (Novato, CA), and many others.

Purification of the primers and probes of the present disclosure, where necessary or desired, may be carried out by any of a variety of methods well-known in the art. Purification of primers and probes can be performed either by native acrylamide gel electrophoresis, by anion-exchange HPLC as described, for example, by Pearson et al., J. Chrom., 1983, 255: 137-149 or by reverse phase HPLC (See, McFarland et al., Nucleic Acids Res., 1979, 7: 1067-1080).

As previously mentioned, modified primers and probes may be prepared using any of several means known in the art. Non-limiting examples of such modifications include methylation, substitution of one or more of the naturally occurring nucleotides with an analog, and internucleotide modifications such as, for example, those with uncharged linkages (e.g., methyl phosphonates, phosphotriesters, phosphoroamidates, carbamates, etc.), or charged linkages (e.g., phosphorothioates, phosphorodithioates, etc.). Primers and probes may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., to chelate metals, radioactive metals, oxidative metals, etc.), and alkylators. Primers and probes may also be derivatized by formation of a methyl or ethyl phosphotriester or an alkyl phosphoramidate linkage. Furthermore, primers and/or probes of the present disclosure may be modified with a detectable label.

As discussed briefly previously herein, in some embodiments, the primers and/or the probes may be labeled with a detectable label or moiety before being used in one or more amplification/detection methods. Preferably, for use in the methods described herein, one or more probes are labeled with a detectable label or moiety. The role of a detectable label is to allow visualization and/or detection of amplified target sequences (e.g., amplicons). Preferably, the detectable label is selected such that it generates a signal which can be measured and whose intensity is related (e.g., proportionally) to the amount of amplification product in the test sample being analyzed.

The association between one or more labeled probes and the detectable label can be covalent or non-covalent. Labeled probes can be prepared by incorporation of, or conjugation to, a detectable moiety. Labels can be attached directly to the nucleic acid sequence or indirectly (e.g., through a linker). Linkers or spacer arms of various lengths are known in the art and are commercially available, and can be selected to reduce steric hindrance, or to confer other useful or desired properties to the resulting labeled molecules (See, for example, Mansfield et al., Mol. Cell. Probes, 1995, 9: 145-156).

Methods for labeling oligonucleotides, such as primers and/or probes, are well-known to those skilled in the art. Reviews of labeling protocols and label detection techniques can be found in, for example, L. J. Kricka, Ann. Clin. Biochem., 2002, 39: 114-129; van Gijlswijk et al, Expert Rev. Mol. Diagn., 2001, 1: 81-91; and Joos et al, J. Biotechnol., 1994, 35: 135- 153. Standard nucleic acid labeling methods include: incorporation of radioactive agents, direct attachments of fluorescent dyes (See, Smith et al., Nucl. Acids Res., 1985, 13: 2399- 2412) or enzymes (See, Connoly et al., Nucl. Acids. Res., 1985, 13: 4485-4502); chemical modifications of nucleic acid molecules rendering them detectable immunochemically or by other affinity reactions (See, Broker et al., Nucl. Acids Res., 1978, 5: 363-384; Bayer et al., Methods of Biochem. Analysis, 1980, 26: 1-45; Langer et al., Proc. Natl. Acad. Sci. USA, 1981, 78: 6633-6637; Richardson et al., Nucl. Acids Res., 1983, 11 : 6167-6184; Brigati et al., Virol., 1983, 126: 32-50; Tchen et al., Proc. Natl. Acad. Sci., USA, 1984, 81 : 3466-3470; Landegent et al., Exp. Cell Res., 1984, 15: 61-72; and A. H. Hopman et al., Exp. Cell Res., 1987, 169: 357-368); and enzyme-mediated labeling methods, such as random priming, nick translation, PCR, and tailing with terminal transferase (For a review on enzymatic labeling, see, for example, Temsamani et al., Mol. Biotechnol., 1996, 5: 223-232). Any of a wide variety of detectable labels can be used in the present disclosure.

Suitable detectable labels include, but are not limited to, various ligands, radionuclides or radioisotopes (e.g., ³²P, ³⁵S, ³H, ¹⁴C, ¹¹⁵1, ¹³¹I, and the like); fluorescent dyes; chemiluminescent agents (e.g., acridinium esters, stabilized dioxetanes, and the like); spectrally resolvable inorganic fluorescent semiconductor nanocrystals (e.g., quantum dots), metal nanoparticles (e.g., gold, silver, copper and platinum) or nanoclusters; enzymes (e.g., horseradish peroxidase, beta-galactosidase, luciferase, alkaline phosphatase); colorimetric labels (e.g., dyes, colloidal gold, and the like); magnetic labels (e.g., Dynabeads^{™}); and biotin and dioxigenin, or other haptens and proteins for antisera or monoclonal antibodies are available. In certain embodiments, the contemplated probes are fluorescently labeled.

Numerous known fluorescent labeling moieties of a wide variety of chemical structures and physical characteristics are suitable for use in the practice of this disclosure. Suitable fluorescent dyes include, but are not limited to, Quasar^{®} dyes available from Biosearch Technologies, Novato, CA), fluorescein and fluorescein dyes (e.g., fluorescein isothiocyanine (FITC), naphthofluorescein, 4',5'-dichloro-2',7'-dimethoxy-fluorescein, 6-carboxyfluoresceins (e.g., FAM), VIC, NED, carbocyanine, merocyanine, styryl dyes, oxonol dyes, phycoerythrin, erythrosin, eosin, rhodamine dyes (e.g., carboxytetramethylrhodamine or TAMRA, carboxyrhodamine 6G, carboxy-X-rhodamine (ROX), lissamine rhodamine B, rhodamine 6G, rhodamine Green, rhodamine Red, tetramethylrhodamine or TMR), coumarin and coumarin dyes (e.g., methoxycoumarin, dialkylaminocoumarin, hydroxycoumarin and aminomethylcoumarin or AMCA), Oregon Green Dyes (e.g., Oregon Green 488, Oregon Green 500, Oregon Green 514), Texas Red, Texas Red-X, Spectrum Red^{™}, Spectrum Green^{™}, cyanine dyes (e.g., Cy-3^{™}, Cy-5^{™}, Cy-3.5^{™}, Cy-5.5^{™}), Alexa Fluor dyes (e.g., Alexa Fluor 350, Alexa Fluor 488, Alexa Fluor 532, Alexa Fluor 546, Alexa Fluor 568, Alexa Fluor 594, Alexa Fluor 633, Alexa Fluor 660 and Alexa Fluor 680), BODIPY dyes (e.g., BODIPY FL, BODIPY R6G, BODIPY TMR, BODIPY TR, BODIPY 530/550, BODIPY 558/568, BODIPY 564/570, BODIPY 576/589, BODIPY 581/591, BODIPY 630/650, BODIPY 650/665), IRDyes (e.g., IRD40, IRD 700, IRD 800), and the like. Examples of other suitable fluorescent dyes that can be used and methods for linking or incorporating fluorescent dyes to oligonucleotides, such as probes, can be found in RP Haugland, *"*The Handbook of Fluorescent Probes and Research Chemicals", Publisher, Molecular Probes, Inc., Eugene, Oreg. (June 1992)). Fluorescent dyes, as well as labeling kits, are commercially available from, for example, Amersham Biosciences, Inc. (Piscataway, N. J.), Molecular Probes Inc. (Eugene, OR), and New England Biolabs Inc. (Beverly, MA). Rather than being directly detectable themselves, some fluorescent groups (donors) transfer energy to another fluorescent group (acceptor) in a process of fluorescence resonance energy transfer (FRET), and the second group produces the detectable fluorescent signal. In these embodiments, the probe may, for example, become detectable when hybridized to an amplified target sequence. Examples of FRET acceptor/donor pairs suitable for use in the present disclosure include, for example, fluorescein/tetramethylrhodamine, IAEDANS/FITC, IAEDANS/5-(iodoacetomido)fluorescein, B-phycoerythrin/Cy-5, and EDANS/Dabcyl, among others.

FRET pairs also include the use of physically-linked fluorescent reporter/quencher pairs. For example, a detectable label and a quencher moiety may be individually attached to either the 5' end or the 3' end of a probe, therefore placing the detectable label and the quencher moiety at opposite ends of the probe, or apart from one another along the length of the probe. During such time as the probe is not bound to its target sequence, the detectable label and quencher moiety are reversibly maintained within such proximity that the quencher blocks the detection of the detectable label. Upon binding of the probe to a target sequence, the detectable label and quencher moiety are separated thus permitting detection of the detectable label under appropriate conditions.

The use of such systems in TaqMan^{®} assays (as described, for example, in U.S. Patent Nos. 5,210,015, 5,804,375, 5,487,792, and 6,214,979) or as Molecular Beacons (as described, for example in, Tyagi et al, Nature Biotechnol., 1996, 14: 303-308; Tyagi et al, Nature Biotechnol., 1998, 16: 49-53; Kostrikis et al., Science, 1998, 279: 1228-1229; Sokol et al., Proc. Natl Acad. Sci. USA, 1998, 95: 11538-11543; Marras et al., Genet. Anal., 1999, 14: 151-156; and U.S. Patent Nos. 5,846,726, 5,925,517, 6,277,581 and 6,235,504) is well-known to those skilled in the art. With the TaqMan^{®} assay format, products of the amplification reaction can be detected as they are formed in a "real-time" manner: amplification product/probe hybrids are formed and detected while the reaction mixture is under amplification conditions.

In some embodiments of the present disclosure, the PCR detection probes are TaqMan^{®}-like probes that are labeled at the 5 '-end with a fluorescent moiety and at the 3'- end with a quencher moiety or alternatively the fluorescent moiety and quencher moiety are in reverse order, or further they may be placed along the length of the sequence to provide adequate separation when the probe hybridizes to a target sequence to allow satisfactory detection of the fluorescent moiety. Suitable fluorophores and quenchers for use with TaqMan^{®} -like probes are disclosed in U.S. Patent Nos. 5,210,015, 5,804,375, 5,487,792, and 6,214,979, and WO 01/86001. Examples of quenchers include, but are not limited, to DABCYL (e.g., 4-(4'-dimethylaminophenylazo)-benzoic acid) succinimidyl ester, diarylrhodamine carboxylic acid, succinimidyl ester (or QSY-7), and 4',5'-dinitrofluorescein carboxylic acid, succinimidyl ester (or QSY-33) (all of which are available from Molecular Probes (which is part of Invitrogen, Carlsbad, CA)), quencher 1 (Ql; available from Epoch Biosciences, Bothell, WA), or "Black hole quenchers" BHQ-I, BHQ-2, and BHQ-3 (available from BioSearch Technologies, Inc., Novato, CA). In certain embodiments, the PCR detection probes are TaqMan^{®} -like probes that are labeled at the 5' end with FAM and at the 3' end with a Black Hole Quencher^{®} or Black Hole Quencher^{®} plus (Biosearch Technologies, Novato, CA).

A "tail" of normal or modified nucleotides can also be added to probes for detectability purposes. A second hybridization with nucleic acid complementary to the tail and containing one or more detectable labels (such as, for example, fluorophores, enzymes, or bases that have been radioactively labeled) allows visualization of the amplicon/probe hybrids.

The selection of a particular labeling technique may depend on the situation and may be governed by several factors, such as the ease and cost of the labeling method, spectral spacing between different detectable labels used, the quality of sample labeling desired, the effects of the detectable moiety on the hybridization reaction (e.g., on the rate and/or efficiency of the hybridization process), the nature of the amplification method used, the nature of the detection system, the nature and intensity of the signal generated by the detectable label, and the like.

### 3. Methods of Detecting SFTSV in Samples

In some embodiments, provided herein are methods of detecting SFTSV in a sample, such as a biological sample. The methods described herein can be used to determine if a subject is suffering from one or more signs or symptoms related to SFTSV infection.

In some embodiments, provided herein are methods of detecting SFTSV in a sample, comprising contacting the sample with at least one primer, at least one probe, or at least one primer and at least one probe. In some embodiments, the methods are performed using any type of nucleic acid amplification such as PCR. In some embodiments, the methods are performed using fluorescence in-situ hybridization (FISH). For example, the primer(s) and/or probe(s) may be suitable for PCR or FISH techniques. The at least one primer, the at least one probe or the at least one primer and at least one probe may be labeled with at least one detectable label. In some embodiments, the at least one primer, the at least one probe or the at least one primer and at least one probe is derived from an SFTSV polynucleotide, or a complement thereof.

The methods comprise contacting the sample with any suitable combination of primers and probes as described herein. In some aspects, the present disclosure provides methods for detecting the presence of SFTSV in a sample. In other aspects, SFTSV levels may be quantified per test sample by comparing test sample detection values against standard curves generated using serial dilutions of previously quantified suspensions of SFTSV.

In some embodiments, the method of detecting SFTSV in a sample comprises contacting the sample with one or more of the compositions described previously herein. For example, the method may comprise contacting the sample with one or more primer sets and probes as described herein. For example, the method may comprise contacting the sample with at least (a) one forward primer; (b) at least one reverse primer; (c) at least one probe; or (d) any combination of (a)-(c) which are: (i) derived from SEQ ID NO: 1-12, or fragments thereof; or (ii) a complement derived from SEQ ID NO: 1-12, or fragments thereof. Any or all of the at least one forward primer, at least one reverse primer, at least one probe, or any combination thereof, may be labeled with one or more detectable labels.

In some embodiments, the method may comprise contacting the sample with one or more primer sets and one or more probes suitable for SFTSV in a sample. For example, the method may comprise contacting the sample with at least one forward primer having a sequence with at least 80% sequence identity (e.g., 80%, 85%, 90%, 91%, 92%, 93%, 93%, 95%, 96%, 97%, 98%, 99%, or 100%) to SEQ ID NO: 1, SEQ ID NO: 6, SEQ ID NO: 9 or a complement thereof, at least one reverse primer having a sequence with at least 80% sequence identity (e.g., 80%, 85%, 90%, 91%, 92%, 93%, 93%, 95%, 96%, 97%, 98%, 99%, or 100%) to SEQ ID NO: 2, SEQ ID NO: 3, SEQ ID NO: 7, SEQ ID NO: 9, or a complement thereof, and at least one probe having a sequence with at least 80% sequence identity (e.g., 80%, 85%, 90%, 91%, 92%, 93%, 93%, 95%, 96%, 97%, 98%, 99%, or 100%) to SEQ ID NO: 4, SEQ ID NO: 5, SEQ ID NO: 8, SEQ ID NO: 11, SEQ ID NO: 12, or a complement thereof.

In some embodiments, the method of detecting SFTSV in a sample comprises contacting the sample with at least one primer set for detecting SFTSV. For example, in some aspects, the method may comprise contacting the sample with at least one forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof and at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof.

In some aspects, the method may comprise contacting the sample with at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 6 or a complement thereof and at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 7.

In some aspects, the method may comprise contacting the sample with at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 9 or a complement thereof and at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 10 or a complement thereof.

In some aspects, the method of detecting SFTSV in a sample involves contacting the sample with at least two sets of primers, such as a first set of primers and a second set of primers. In one aspect, the first set of primers comprises at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof and at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof. The second set of primers comprises at least one second forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 6 or a complement thereof and at least one second reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 7 or a complement thereof.

In some aspects, the method of detecting SFTSV in a sample involves contacting the sample with at least two sets of primers, such as a first set of primers and a second set of primers. In one aspect, the first set of primers comprises at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof and at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof. The second set of primers comprises at least one second forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 9 or a complement thereof and at least one second reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 10 or a complement thereof.

In some aspects, the method of detecting SFTSV in a sample involves contacting the sample at least three sets of primers, such as a first set of primers, a second set of primers, and a third set of primers. In one aspect, the first set of primers comprises at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof and at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof. The second set of primers comprises at least one second forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 6 or a complement thereof and at least one second reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 7 or a complement thereof. The third set of primers comprises at least one third forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 9 or a complement thereof and at least one third reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 10 or a complement thereof.

In some embodiments, the method of detecting SFTSV in a sample comprises contacting the sample with at least one primer set and at least one probe for detecting SFTSV. For example, in some aspects, the method may comprise contacting the sample with at least one forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof, at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof, and at least one first probe having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 4 and/or 5 or a complement thereof.

In some aspects, the method may comprise contacting the sample with at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 6 or a complement thereof, at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 7 or a complement thereof and at least one probe having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 8 or a complement thereof.

In some aspects, the method may comprise contacting the sample with at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 9 or a complement thereof, at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 10 or a complement thereof, and at least one first probe having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 11 and/or 12 or a complement thereof

In some aspects, the method of detecting SFTSV in a sample involves contacting the sample with at least two sets of primers and at least two probes, such as a first set of primers and a first probe and a second set of primers and a second probe. In one aspect, the first set of primers comprises at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof and at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof and a first probe having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 4 or 5 or a complement thereof. The second set of primers comprises at least one second forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 6 or a complement thereof and at least one second reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 7 or a complement thereof and a second probe having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 8 or a complement thereof.

In some aspects, the method of detecting SFTSV in a sample involves contacting the sample with at least two sets of primers and at least two probes, such as a first set of primers and a first probe and a second set of primers and a second probe. In one aspect, the first set of primers comprises at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof and at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof and a first probe having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 4 and/or 5 or a complement thereof. The second set of primers comprises at least one second forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 9 or a complement thereof and at least one second reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 10 or a complement thereof and a second probe having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 11 and/or 12 or a complement thereof.

In some aspects, the method of detecting SFTSV in a sample involves contacting the sample at least three sets of primers, such as a first set of primers, a second set of primers, and a third set of primers and at least three probes. In one aspect, the first set of primers comprises at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof and at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof and at least one first probe having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 4 and/or 5 or a complement thereof. The second set of primers comprises at least one second forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 6 or a complement thereof and at least one second reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 7 or a complement thereof and a second probe having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 8 or a complement thereof. The third set of primers comprises at least one third forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 9 or a complement thereof and at least one third reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 10 or a complement thereof and a third probe having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 11 and/or 12 or a complement thereof.

The presence or absence of the amplified products is detected using routine techniques known in the art. In some exemplary embodiments, a real time probe displacement assay as described above is utilized. Further, as described herein, the assays of the present disclosure utilize detection of both L and S segments of SFSTV, including lineage I and lineage II of the S segment. Specifically, if at least two amplified products are detected, this indicates that SFSTV is present in the sample. If two amplified products are not detected in the sample (e.g., one or no amplified products are detected), this indicates that SFSTV may not be present in the sample. In addition, in other aspects, the above method also includes including a third primer set and a third probe to determine if the S segment is present. The use of two different targets (e.g. lineage I and lineage II) on the S segment helps to avoid mutation escape.

In some embodiments, all three sets of primers and probes are utilized in a single reaction mixture for multiplexing reactions. In some embodiments, probes for detection of the L segment have a different detectable label than those for detecting the S segment.

The amplification conditions may comprise submitting the sample to an amplification reaction carried out in the presence of suitable amplification reagents. In some embodiments, the amplification reaction comprises PCR, real-time PCR, or reverse-transcriptase PCR.

The use of primers, probes, primers and probes, primer sets, or primer sets and probe sets of the present disclosure to amplify SFSTV target sequences in test samples is not limited to any particular nucleic acid amplification technique or any particular modification thereof. In fact, the primers, probes, primer sets, and primer sets and probes of the present disclosure can be employed in any of a variety of nucleic acid amplification methods that are known in the art (See, for example, Kimmel et al., Methods Enzymol., 1987, 152: 307-316; Sambrook et al., "Molecular Cloning. A Laboratory Manual", 1989, 2.Supp. Ed., Cold Spring Harbour Laboratory Press: New York, NY; *"*Short Protocols in Molecular Biology", F. M. Ausubel (Ed.), 2002, 5. Supp. Ed., John Wiley & Sons: Secaucus, NJ).

Such nucleic acid amplification methods include, but are not limited to, the Polymerase Chain Reaction (PCR). PCR is described in a number of references, such as, but not limited to, *"*PCR Protocols: A Guide to Methods and Applications", M. A. Innis (Ed.), 1990, Academic Press: New York; *"*PCR Strategies", M. A. Innis (Ed.), 1995, Academic Press: New York; *"*Polymerase chain reaction: basic principles and automation in PCR. A Practical Approach", McPherson et al. (Eds.), 1991, IRL Press: Oxford; Saiki et al., Nature, 1986, 324: 163; and U.S. Patent Nos. 4,683,195, 4,683,202 and 4,889,818. Variations of PCR including, TaqMan^{®} -based assays (See, Holland et al., Proc. Natl. Acad. Sci., 1991, 88: 7276-7280), and reverse transcriptase polymerase chain reaction (or RT-PCR, described in, for example, U.S. Patent Nos. 5,322,770 and 5,310,652) are also included.

Generally, in PCR, at least one pair of primers is added to a test sample obtained from a subject (and thus contacted with the test sample) in excess to hybridize to the complementary strands of the target nucleic acid. The primers are each extended by a DNA polymerase using the target sequence as a template. The extension products become targets themselves after dissociation (denaturation) from the original target strand. New primers are then hybridized and extended by the polymerase, and the cycle is repeated to exponentially increase the number of amplicons. Examples of DNA polymerases capable of producing primer extension products in PCR reactions include, but are not limited to, E. coli DNA polymerase I, Klenow fragment of DNA polymerase I, T4 DNA polymerase, thermostable DNA polymerases isolated from Thermus aquaticus (Taq), available from a variety of sources (e.g., Perkin Elmer, Waltham, MA), Thermus thermophilus (USB Corporation, Cleveland, OH), Bacillus stereothermophilus (Bio-Rad Laboratories, Hercules, CA), AmpliTaq Gold^{®} Enzyme (Applied Biosystems, Foster City, CA), recombinant Thermus thermophilus (rTth) DNA polymerase (Applied Biosystems, Foster City, CA) or Thermococcus litoralis ("Vent" polymerase, New England Biolabs, Ipswich, MA). RNA target sequences may be amplified by first reverse transcribing (RT) the mRNA into cDNA, and then performing PCR (RT- PCR), as described above. Alternatively, a single enzyme may be used for both steps as described in U.S. Patent No. 5,322,770.

In addition to the enzymatic thermal amplification methods described above, isothermal enzymatic amplification reactions can be employed to amplify SFSTV using primers and primer sets of the present disclosure (Andras et al., Mol. Biotechnol., 2001, 19: 29-44). These methods include, but are not limited to, Transcription-Mediated Amplification (TMA; TMA is described in Kwoh et al., Proc. Natl. Acad. Sci USA, 1989, 86: 1173-1177; Giachetti et al., J. Clin. Microbiol, 2002, 40: 2408-2419; and U.S. Patent No. 5,399,491); Self-Sustained Sequence Replication (3SR; 3SR is described in Guatelli et al., Proc. Natl. Acad. Sci. USA, 1990, 87: 1874-1848; and Fahy et al., PCR Methods and Applications, 1991, 1: 25-33); Nucleic Acid Sequence Based Amplification (NASBA; NASBA is described in, Kievits et al., J. Virol. Methods, 1991, 35: 273-286; and U.S. Patent No. 5,130,238) and Strand Displacement Amplification (SDA; SDA is described in Walker et al., PNAS, 1992, 89: 392-396; EP 0 500 224 A2).

In certain embodiments of the present disclosure, the one or more probes described herein are used to detect amplification products generated by the amplification reaction. The probes described herein may be employed using a variety of well-known homogeneous or heterogeneous methodologies.

Homogeneous detection methods include, but are not limited to, the use of FRET labels that are attached to the probes and that emit a signal in the presence of the target sequence, Molecular Beacons (See, Tyagi et al., Nature Biotechnol., 1996, 14: 303-308; Tyagi et al., Nature Biotechnol., 1998, 16: 49-53; Kostrikis et al., Science, 1998, 279: 1228- 1229; Sokol et al., Proc. Natl. Acad. Sci. USA, 1998, 95: 11538-11543; Marras et al., Genet. Anal., 1999, 14: 151-156; and U.S. Patent Nos. 5,846,726, 5,925,517, 6,277,581 and 6,235,504), and the TaqMan^{®} assays (See, U.S. Patent Nos. 5,210,015; 5,804,375; 5,487,792 and 6,214,979 and WO 01/86001). Using these detection techniques, products of the amplification reaction can be detected as they are formed, namely, in a real time manner. As a result, amplification product/probe hybrids are formed and detected while the reaction mixture is under amplification conditions.

In certain embodiments, the probes of the present disclosure are used in a TaqMan^{®} assay. In a TaqMan^{®} assay, analysis is performed in conjunction with thermal cycling by monitoring the generation of fluorescence signals. The assay system has the capability of generating quantitative data allowing the determination of target copy numbers. For example, standard curves can be generated using serial dilutions of previously quantified suspensions of SFSTV against which unknown samples can be compared. The TaqMan^{®} assay is conveniently performed using, for example, AmpliTaq Gold^{™} DNA polymerase, which has endogenous 5' nuclease activity, to digest a probe labeled with both a fluorescent reporter dye and a quencher moiety, as described above. Assay results are obtained by measuring changes in fluorescence that occur during the amplification cycle as the probe is digested, uncoupling the fluorescent and quencher moieties and causing an increase in the fluorescence signal that is proportional to the amplification of the target sequence.

Other examples of homogeneous detection methods include hybridization protection assays (HPA). In such assays, the probes are labeled with acridinium ester (AE), a highly chemiluminescent molecule (See, Weeks et al, Cln. Chem., 1983, 29: 1474-1479; Berry et al., Cln. Chem., 1988, 34: 2087-2090), using a non-nucleotide-based linker arm chemistry (See, U.S. Patent Nos. 5,585,481 and 5,185,439). Chemiluminescence is triggered by AE hydrolysis with alkaline hydrogen peroxide, which yields an excited N-methyl acridone that subsequently deactivates with emission of a photon. In the absence of a target sequence, AE hydrolysis is rapid. However, the rate of AE hydrolysis is greatly reduced when the probe is bound to the target sequence. Thus, hybridized and un-hybridized AE-labeled probes can be detected directly in solution without the need for physical separation.

Heterogeneous detection systems are also well-known in the art and generally employ a capture agent to separate amplified sequences from other materials in the reaction mixture. Capture agents typically comprise a solid support material (e.g., microtiter wells, beads, chips, and the like) coated with one or more specific binding sequences. A binding sequence may be complementary to a tail sequence added to oligonucleotide probes of the disclosure. Alternatively, a binding sequence may be complementary to a sequence of a capture oligonucleotide, itself comprising a sequence complementary to a tail sequence of a probe. After separation of the amplification product/probe hybrids bound to the capture agents from the remaining reaction mixture, the amplification product/probe hybrids can be detected using any detection methods, such as those described herein.

In some embodiments, the methods further comprise administering an appropriate therapy to the subject if SFTSV is detected in the sample. For example, the method may further comprise administering an appropriate anti-viral agent to the subject. In some aspects, the anti-viral agent may be ribavirin, favipiravir, or fludarabine.

### 4. Kits

In another embodiment, the present disclosure provides kits including materials and reagents useful for the detection of SFTSV. The description of the primers, probes, and compositions herein are also applicable to those same aspects of the methods for detecting SFTSV. The kits can be used by diagnostic laboratories, experimental laboratories, or practitioners. In certain embodiments, the kits comprise at least one of primers, probes, primer sets, or primer sets and probes described in herein and optionally, amplification reagents. Each kit preferably comprises amplification reagents for a specific amplification method. Thus, a kit adapted for use with NASBA preferably contains primers with an RNA polymerase promoter linked to the target binding sequence, while a kit adapted for use with SDA preferably contains primers including a restriction endonuclease recognition site 5' to the target binding sequence. Similarly, when the kit is adapted for use in a 5' nuclease assay, such as the TaqMan^{®} assay, the probes of the present disclosure can contain at least one fluorescent reporter moiety and at least one quencher moiety.

In some embodiments, the kit comprises at least one forward primer, at least one reverse primer, at least one probe, and amplification reagents and instructions for amplifying and detecting SFTSV. Any of the primers, probes or primers and probes contained in kit may comprise a detectable label and/or quencher.

In some embodiments, the kit comprises at least one forward primer, at least one reverse primer, and at least one probe which can be: (i) derived from SEQ ID NOs: 1-12 or fragments thereof; or (ii) a complement derived from SEQ ID NOs: 1-12 or fragments thereof. The kit may comprise one forward primer or more than one (e.g., 2, 3, 4, or more) forward primers. The kit may comprise one reverse primer or more than one (e.g. 2, 3, 4, 5, or more) reverse primers. The kit may comprise one probe or more than one (e.g., 2, 3, 4, 5, or more) probes. Any one or more primers and/or probes may be labeled with a detectable label.

In some embodiments, the kit comprises at least one forward primer having a sequence with at least 80% sequence identity (e.g., 80%, 81, 82%, 83%, 84%, 85%, 86%, 87%, 88%, 89% 90%, 91%, 92%, 93%, 93%, 95%, 96%, 97%, 98%, 99%, or 100%) to SEQ ID NO: 1, SEQ ID NO: 6, SEQ ID NO: 9, or complements thereof, at least one reverse primer having a sequence with at least 80% sequence identity (e.g., 80%, 81, 82%, 83%, 84%, 85%, 86%, 87%, 88%, 89% 90%, 91%, 92%, 93%, 93%, 95%, 96%, 97%, 98%, 99%, or 100%) to SEQ ID NO: 2, SEQ ID NO: 3, SEQ ID NO: 7, SEQ ID NO: 10, or complements thereof. The kit may further comprise at least one probe having a sequence with at least 80% sequence identity (e.g., 80%, 81, 82%, 83%, 84%, 85%, 86%, 87%, 88%, 89% 90%, 91%, 92%, 93%, 93%, 95%, 96%, 97%, 98%, 99%, or 100%to SEQ ID NO: 4, SEQ ID NO: 5, SEQ ID NO: 8, SEQ ID NO: 11, SEQ ID NO; 12, or complements thereof. The kit may comprise one forward primer or more than one (e.g., 2, 3, 4, or more) forward primers. The kit may comprise one reverse primer or more than one (e.g., 2, 3, 4, 5, or more) reverse primers. The kit may comprise one probe or more than one (e.g., 2, 3, 4, 5, or more) probes. Any one or more primers, probers, or primers and probes may be labeled with a detectable label.

In some embodiments, the kit comprises at least one forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof, at least one reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof, and at least one probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 4 and/or 5, or a complement thereof. In the kit, the forward primer of SEQ ID NO: 1 or a complement thereof, the reverse primer of SEQ ID NO: 2 and/or 3 or a complement thereof, and the probe of SEQ ID NO: 4 and/or 5 or a complement thereof can be used to detect the L segment of SFTSV. Additionally, in the kit the at least one forward primer, the at least one reverse primer, the at least one probe, or any combination thereof can be labeled with one or more detectable labels.

In some embodiments, the kit comprises at least one forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 6 or a complement thereof, at least one reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 7 or a complement thereof, and at least one probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 8, or a complement thereof. In the kit, the forward primer of SEQ ID NO: 6 or a complement thereof, the reverse primer of SEQ ID NO: 7 or a complement thereof, and the probe of SEQ ID NO: 8 or a complement thereof can be used to detect the S segment of SFTSV. Additionally, in the kit the at least one forward primer, the at least one reverse primer, the at least one probe, or any combination thereof can be labeled with one or more detectable labels.

In some embodiments, the kit comprises at least one forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 9 or a complement thereof, at least one reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 10 or a complement thereof, and at least one probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 11 and/or 12 or a complement thereof. In the kit, the forward primer of SEQ ID NO: 9 or a complement thereof, the reverse primer of SEQ ID NO: 10 or a complement thereof, and the probe of SEQ ID NO: 11 and/or 12 or a complement thereof can be used to detect the S segment of SFTSV. Additionally, in the kit the at least one forward primer, the at least one reverse primer, the at least one probe, or any combination thereof can be labeled with one or more detectable labels.

In some embodiments, the kit comprises at least two sets of primers and at least two probes, such as a first set of primers and a first probe and a second set of primers and a second probe. In one aspect, the first set of primers comprises at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof, at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof, and at least one first probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 4 and/or 5, or a complement thereof. The second set of primers comprises at least one second forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 6 or a complement thereof, at least one second reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 7 or a complement thereof, and at least one second probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 8, or a complement thereof. In the kit, the forward primers of SEQ ID NO: 1, SEQ ID NO: 6 or a complement thereof, the reverse primers of SEQ ID NO: 2, SEQ ID NO: 3, SEQ ID NO: 7 or a complement thereof, and the probes of SEQ ID NO: 4, SEQ ID NO: 5, SEQ ID NO: 8 or a complement thereof, can be used to detect the S and L segments of SFTSV. Additionally, in the kit the at least one first forward primer, the at least one first reverse primer, the at least one first probe, the at least one second forward primer, the at least one second reverse primer, the at least one second probe, or any combination thereof can be labeled with one or more detectable labels.

In some embodiments, the kit comprises at least two sets of primers and at least two probes, such as a first set of primers and a first probe and a second set of primers and a second probe. In one aspect, the first set of primers comprises at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof, at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof, and at least one first probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 4 and/or 5, or a complement thereof. The second set of primers comprises at least one second forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 9 or a complement thereof, at least one second reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 10 or a complement thereof, and at least one second probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 11 and/or 12, or a complement thereof. In the kit, the forward primers of SEQ ID NO: 1, SEQ ID NO: 9 or a complement thereof, the reverse primers of SEQ ID NO: 2, SEQ ID NO: 3, SEQ ID NO: 10 or a complement thereof, and the probes of SEQ ID NO: 4, SEQ ID NO: 5, SEQ ID NO: 11, SEQ ID NO; 12 or a complement thereof, can be used to detect the S and L segments of SFTSV. Additionally, in the kit the at least one first forward primer, the at least one first reverse primer, the at least one first probe, the at least one second forward primer, the at least one second reverse primer, the at least one second probe, or any combination thereof can be labeled with one or more detectable labels.

In some embodiments, the kit comprises at least three sets of primers, such as a first set of primers, a second set of primers, and a third set of primers. In one aspect, the first set of primers comprises at least one first forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 1 or a complement thereof, at least one first reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 2 and/or 3 or a complement thereof, and at least one first probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 4 and/or 5, or a complement thereof. The second set of primers comprises at least one second forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 6 or a complement thereof, at least one second reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 7 or a complement thereof, and at least one second probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 8, or a complement thereof. The third set of primers comprises at least one third forward primer having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 9 or a complement thereof, at least one third reverse primer having a sequence with at least 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 10 or a complement thereof, and at least one third probe having a sequence with at least 80% sequence identity (e.g., 80%, 81%, 82%, 83%, 84%, 85%, 86%, 87%, 88%, 89%, 90%, 91%, 92%, 93%, 94%, 95%, 96%, 97%, 98%, 99%, or 100%) to SEQ ID NO: 11 and/or 12, or a complement thereof. In the kit, the forward primers of SEQ ID NO: 1, SEQ ID NO: 7, SEQ ID NO: 9, or a complement thereof, the reverse primers of SEQ ID NO: 2, SEQ ID NO: 3, SEQ ID NO: 7, SEQ ID NO: 9, or a complement thereof, and the probes of SEQ ID NO: 4, SEQ ID NO: 5, SEQ ID NO:8, SEQ ID NO: 11, SEQ ID NO: 12, or a complement thereof, can be used to detect all the S and L segments of SFTSV. Additionally, in the kit the at least one first forward primer, the at least one first reverse primer, the at least one first probe, the at least one second forward primer, the at least one second reverse primer, the at least one second probe, at least one third forward primer, at least one third reverse primer, the at least one third probe, or any combination thereof can be labeled with one or more detectable labels.

As mentioned, the above kits may contain one or more suitable amplification reagents. Suitable amplification reagents additionally include, for example, one or more of: buffers, reagents, enzymes having reverse transcriptase and/or polymerase activity or exonuclease activity, enzyme cofactors such as magnesium or manganese; salts; deoxynucleotide triphosphates (dNTPs) suitable for carrying out the amplification reaction. Depending on the procedure, kits may further comprise one or more of: wash buffers, hybridization buffers, labeling buffers, detection means, and other reagents. The buffers and/or reagents are preferably optimized for the particular amplification/detection technique for which the kit is intended. Protocols for using these buffers and reagents for performing different steps of the procedure may also be included in the kit. Furthermore, kits may be provided with an internal control as a check on the amplification efficiency, to prevent occurrence of false negative test results due to failures in the amplification, to check on cell adequacy, sample extraction, etc. An optimal internal control sequence is selected in such a way that it will not compete with the target nucleic acid sequence in the amplification reaction. Such internal control sequences are known in the art. Kits may also contain reagents for the isolation of nucleic acids from test samples prior to amplification before nucleic acid extraction.

The reagents may be supplied in a solid (e.g., lyophilized) or liquid form. Kits of the present disclosure may optionally comprise different containers (e.g., vial, ampoule, test tube, flask, or bottle) for each individual buffer and/or reagent. Each component will generally be suitable as aliquoted in its respective container or provided in a concentrated form. Other containers suitable for conducting certain steps of the amplification/detection assay may also be provided. The individual containers are preferably maintained in close confinement for commercial sale. Different components of the kits can be provided in one or more containers.

Kits may also comprise instructions for using the amplification reagents and primers, probes, primer sets, or primer sets and probes described herein, such as, for processing the test sample, extracting nucleic acid molecules, and/or performing the test; and for interpreting the results obtained as well as a notice in the form prescribed by a governmental agency. Such instructions optionally may be in printed form or on CD, DVD, or other format of recorded media. By way of example, and not of limitation, examples of the present disclosures shall now be given.

The present disclosure has multiple aspects, illustrated by the following nonlimiting examples.

### Example 1

### Detection of SFTSV

### Materials and Methods

### Ethics Statement

This study received approval from the Institutional Review Board of the Faculty of Medicine at Siriraj Hospital, Thailand, for the use of leftover archived samples and their associated clinical data. (COA Si.391/2021).

### Sample Collection

1,605 plasma samples from in-patients at the Faculty of Medicine Siriraj Hospital, Mahidol University, Bangkok, Thailand presenting with acute undifferentiated febrile illness collected during 2012-2021 were used in this study. A total of n=1,605 were tested using the specific dual targeted RT-qPCR developed for identifying SFTSV, n=1784 were analyzed by metagenomic and target enriched NGS, and n=1,391 were used to evaluate the serological assay.

### Total Nucleic Acid Extraction and cDNA Synthesis

Samples were pre-treated with benzonase (> 250 units/µl, Sigma-Aldrich, St. Louis, MO) for three (3) hours at 37°C to decrease human background nucleic acids (718 µl serum + 80 µl of 10× buffer + 2 µl Benzonase). Samples with less than the required volume were diluted with 1X PBS to obtain the required 718 µl sample volume requirement. Extraction of total nucleic acid (TNA) from 500 µl of the treated sample was performed on the Abbott Diagnostics m2000sp using the Sample Preparation SystemDNA (Abbott Diagnostics, Chicago). Samples were also extracted with a KingFisher instrument (358 µl serum + 40 µl of 10× buffer + 2 µl Benzonase) according to manufacturer (Thermo Fisher Scientific) instructions. The extraction contained a positive control (five viral stocks spiked into HIV-positive plasma at 3.0 log copies/ml) and one negative control (normal human plasma). cDNA was generated from 10 µl of the extracted TNA using the Super-Script IV First-Strand Synthesis System for RT-PCR (Invitrogen, ThermoFisher Scientific) and Sequenase v2.0 DNA Polymerase (Applied Biosystems, ThermoFisher Scientific). The products were cleaned with AMPure XP beads (Beckman Coulter).

### Library Preparation, Target Enrichment and DNA Sequencing

NGS libraries were prepared using the Illumina Nextera XT v2 Library Preparation Kit and amplified for 24 cycles with non-biotinylated, unique dual index barcode adapters (Integrated DNA Technologies). Libraries were purified with AMPure XP beads (Beckman Coulter, California). Libraries were assessed for size and quality using a TapeStation 4200 (Agilent) and quantified using a Qubit Flex Fluorometer (ThermoFisher Scientific). Next, an aliquot of each metagenomic library, with a maximum of twenty-four libraries and 3 000 ng total, were pooled together for target enrichment. Pools were dried using a vacuum centrifuge, resuspended in a solution of human Cot-1 DNA and Universal Nextera Blockers (Integrated DNA Technologies), and hybridized to Com-prehensive Viral Research Panel (CVRP, Twist Biosciences) probes for 16 hours per manufacturer instructions. Hybridized reads were captured by affinity interaction on streptavidin beads (Twist Biosciences), amplified using a KAPA library amplification kit (Roche), and re-purified using magnetic beads (Twist Biosciences). Viral-enriched libraries were analyzed for size and concentration as before. Next generation sequencing of CVRP-enriched libraires was performed on an Illumina MiSeq instrument using a MiSeq v2 300 cycle flow cell. The resulting raw data was processed through an in-house pipeline to identify sequences of human pathogens and imported to Qiagen CLC Genomics Workbench to map the reads to SFTSV reference sequences and generate consensus sequences.

### Genomic data collection and data curation Sequence Alignment and phylogenetic inference

All SFTSV sequences utilized in this study were retrieved from the GenBank database on September 18th, 2024. The methodology described by Perez et al.(Pérez, Baele et al. 2024) was used. Briefly: a deduplication analysis was conducted following the methodology described by Perez et al. (Perez, Cloherty and Berg 2021). Additional details such as the year of collection, country of origin, strain name, and host species were extracted by using the gbmunge script, available at GitHub: https://github.com/sdwfrost/gbmunge, supplemented by a custom R script. In all cases the sequences were aligned using the MAFFT software v7.453 (Rozewicki, Li et al. 2019) with settings for a "localpair" option alignment. The alignments were then utilized for maximum likelihood (ML) phylogenetic inference using IQ-TREE2 (Nguyen, Schmidt et al. 2015). Initially, the ModelFinder algorithm was employed to identify the most appropriate nucleotide substitution model based on the Bayesian Information Criterion. Subsequently, ML phylogenetic trees were constructed. To evaluate the reliability of the branching patterns within the ML trees, 10,000 replicates each of the Shimodaira-Hasegawa Approximate Likelihood Ratio Test and Ultra-fast Bootstrapping were conducted. All visualizations were then performed using custom R scripts.

### Results

### Discovery, NGS, and clinical manifestations

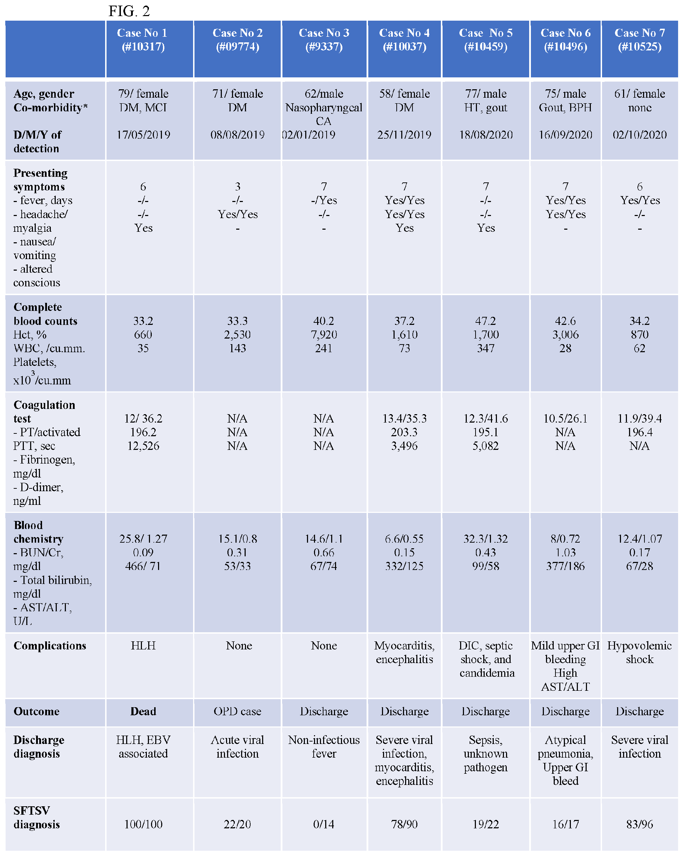

Hospitalized patients (n>3000) at Siriraj Hospital, Bangkok, Thailand were enrolled in a study spanning 2012-2021 to determine etiologies of acute undifferentiated febrile illness. Using metagenomic next-generation sequencing (mNGS), a variety of pathogens were detected in individuals including arboviruses (e.g. Dengue, Chikungunya) and bacteria (e.g. Leptospira, Rickettsia). NGS reads for SFTSV, mapping to each of the three genome segments, were detected in 7 individuals (Table 1; FIG. 2). Dengue NS 1, IgM, IgG markers and antibodies against Rickettsia typhi and Orientia tsutsugamushi were not detected in these patients. Patient presentations were consistent with the known course of SFTSV infection, including fever, myalgia, and complete blood counts (CBC) indicating marked leukopenia and thrombocytopenia (Table 1). The advanced ages (≥ 75 yr) of several patients also reflect those believed to be most at risk. All cases were treated with empirical ceftriaxone and azithromycin for 7 days and their WBC count and platelets were normal prior to discharge. Considering clinical data and supportive diagnostics results discussed later, five NGS+ individuals (10525, 10317, 10459, 10037, 10496) were ruled confirmed cases and two (09774, 09337) were ruled possible infections. The clinical presentation of three confirmed cases of SFTSV are described below.

Subject 10459 was a 77- year- old male with gout and hypertension from Supanburi, central Thailand. He was hospitalized in August of 2020 with 7 days of low-grade fever (37.9°C) and altered consciousness. Laboratory tests revealed a platelet count of 47 × 10⁶ platelets/L and a white blood cell count of 1.79 cells/cu.mm (Table 1). The initial diagnosis was sepsis with disseminated intravascular coagulopathy (DIC). He received packed red cell and platelet transfusion as clinically indicated. He exhibited lower gastrointestinal bleeding, bleeding per tracheal suction during mechanical ventilatory support, and progressive rising of ALT/AST levels to maximum levels of 475/1372 mg/dl at day 4 of admission. Leukopenia resolved after 4 days of admission, but thrombocytopenia persisted for 3 weeks. His abnormal ALT/AST levels were resolved at 4 weeks after the illness. The discharge diagnosis was atypical tropical infection with unknown pathogen. SFTSV NGS coverage was moderate: 19% mNGS, 22% teNGS.

Subject 10525 was a 61- year- old previously healthy, female construction worker who lived in Bangkok and was hospitalized in October 2020 with a diagnosis of dengue hemorrhagic fever (DHF). She had 6 days of high fever (39.2°C) with chills. Laboratory tests revealed a platelet count of 62 × 10³ platelets/L and a white blood cell count of 870 cells/cu.mm (Table 1). She was afebrile after 4 days of admission and white blood cell and platelets levels improved to normal range at 2 weeks following antibiotics. The diagnosis on discharge was acute febrile illness with unknown pathogen. SFTSV NGS coverage was strong: 83% mNGS, 96% teNGS.

Subject 10317 was a 79-year-old female with diabetes mellitus type2 (DM) and mild cognitive impairment (MCI), referred from her local hospital in Kanchanaburi, central Thailand and admitted to Siriraj Hospital in May of 2019 due to persistent high fever (40°C) for 6 days, altered consciousness and progressive leukopenia and thrombocytopenia. She was unaware of having an insect bite. She developed respiratory failure, acute kidney injury and refractory hypotension. Diagnosis of hemophagocytic lymphohistiocytosis (HLH) associated with unknown viral infection (secondary to EBV infection detected by PCR) was made based on leukopenia, thrombocytopenia, and hemophagocytosis found by bone marrow examination. The patient's condition deteriorated rapidly, and she died 4 days after admission to the ICU from multiorgan failure. This is the first documented death from SFTSV in Thailand. SFTSV NGS coverage was strong: 100% mNGS, 100% teNGS.

### Example 2

### Diagnostic Assays for SFTSV

### Materials and Methods

Considering the evolutionary rates of all three segments of SFTSV determined by Perez et al. (Pérez, Baele et al. 2024), as well as the forces driving genetic diversity in this viral species including recombination, purifying selection, and positive selection, target regions within genome segments L and S were selected for target regions (FIG. 3). Thus, the L segment is used to track the virus, and the S segment to monitor the emergence of new strains based on its higher evolutionary rate. Primer and probe sets were selected following the methodology described by Perez et al. (Pérez, Lanka et al. 2020). Briefly, using all available sequences for both segments after a deduplication process, regions of lower variability were selected based on Shannon entropy analysis. Primers and probes were designed using Oligo 7.6 software (Molecular Biology Insights, United States) with selection parameters set to optimize priming efficiency in a TaqMan assay (Acevedo, Perera et al. 2013, Rios, Perera et al. 2018). In silico evaluations for specificity were performed using the GenBank nucleotide database and BLASTn searches.

RT-qPCR experiments were conducted on an m2000rt Real-Time PCR System instrument (Abbott Molecular, Des Plaines, IL, United States), and results were analyzed using MultiAnalyze software. Serial dilutions of in vitro transcripts in nuclease-free water were tested with the respective primer/probe sets to determine linearity and efficiency. Similarly, the analytical sensitivity (ASe) was estimated by evaluating ten-fold serial dilutions of quantified RNA transcript standards, determined using a Probit regression analysis implemented in MedCalc Statistical Software version 19.0.7 (MedCalc Software bvba, Ostend, Belgium; 2019). The ASe was defined as the concentration (copies of the transcripts per reaction) that yielded 95% positive results along with a 95% Confidence Interval (CI) (Pérez, Lanka et al. 2020). Finally, a Research Use Only (RUO) for the automated m2000 platform (Abbott Molecular, Des Plaines, IL) was developed following the chemistry conditions and internal control addition as described in Anderson et al. (Anderson, Hodges et al. 2024).

### Results

To demonstrate this experimentally and to estimate the disease burden among febrile cases in the country, a dual-target RT-qPCR test was used. The literature was first reviewed for previously described assays (Jalal, Hwang et al. 2021). Conventional RT-PCR and nested RT-PCR assays prone to high rates of contamination (Pérez, Lanka et al. 2020) with amplicon sizes larger than 150 bp which reduce sensitivity (Bustin and Nolan 2004) were not considered. An evaluation of real-time RT-qPCR assays (Heo, Yoo et al. 2015) found that while the assay could detect most strains of Lineage 1 SFTSV, there was potential for mutational escape due to 5' mismatches (A/C→T) in the minor groove binder (MGB) probe. Further, the primers and probe of this assay completely fail to hybridize to Lineage 2 strains, which are spreading the quickest across Asia. Considering these shortcomings, an assay was designed that is inclusive of Lineage 2 strains while still ensuring the amplification of both lineages.

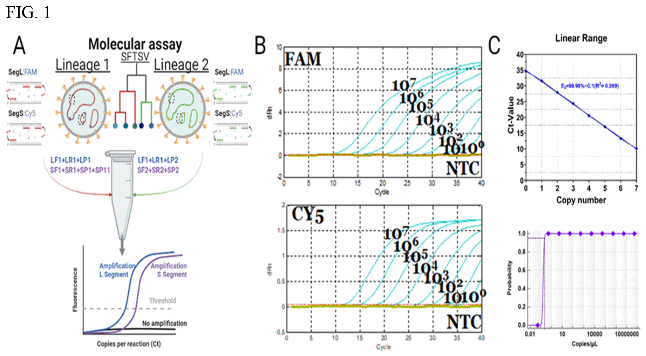

Primer and probe sequences are shown in Figure 3. Both the L (FAM) and S (Cy5) segments (Pérez, Baele et al. 2024) were targeted (Figure 1A) to guarantee the detection of both lineages and serve as a safeguard since the assay simultaneously targets two different segments with distinct evolutionary rates and recombination capabilities.

The conserved L segment encoding RdRp is detected using one forward primer (SEQ ID NO:1) and first and second reverse primers (SEQ ID NOs: 2 and 3) and first and second probes (SEQ ID NOs: 4 and 5) to minimize the number of degenerate bases. During amplification, these redundant primers and probes compete for the target sequence with the closest match in order to maintain the high Tm for extension.

The variable S segment encoding the nucleocapsid, which detects newly emerging strains of SFTSV is detected using two sets of primers and probes to detect multiple lineages. For detection of the first S target (Lineage I), a single forward primer (SEQ ID NO: 9), a single reverse primer (SEQ ID NO: 10), and first and second probes (SEQ ID NOs: 11 and 12) are included.

For detection of the second S target (Lineage II), a single forward primer (SEQ ID NO: 6), a single reverse primer (SEQ ID NO: 7), and a first probe (SEQ ID NO: 8) is included.

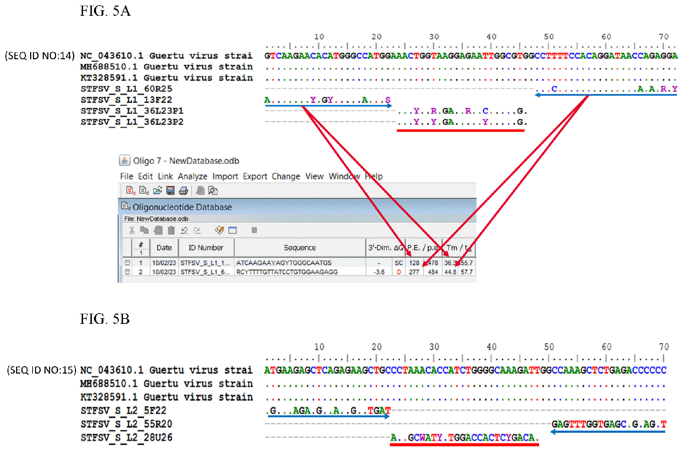

An in silico exclusivity analysis of the assay demonstrated its specificity for SFTSV, as the primers and probe sets failed to hybridize with its closest genetically related viral species, Bandavirus guertuense (Figures 4-5).

The assay was linear over the range spanning 10⁷ to 10° copies per reaction in both channels (FAM and Cy5), with R² values approaching 1.0 and efficiencies of approximately 96% for both segments (Figure 1B). Analytical sensitivity, determined by probit function analysis at a 95% confidence interval measured on 10 replicates per dilution (see Materials and Methods section), estimated a limit of detection of approximately 1 copy per reaction, corresponding to Ct values of 39.3 ±0.3 (Figure 1C). Finally, the assay was converted to Abbott chemistry and transferred to automated platforms where it still yielded the same analytical performance. The SFTSV RT-qPCR was used to screen a cohort of 1,605 AFI patients from Thailand. Three of seven NGS positives were detected, along with 3 additional patients that were NGS negative, for a total of 10 RNA positives.

It is understood that the foregoing detailed description and accompanying examples are merely illustrative and are not to be taken as limitations upon the scope of the disclosure, which is defined solely by the appended claims and their equivalents.

Various changes and modifications to the disclosed embodiments will be apparent to those skilled in the art. Such changes and modifications, including without limitation those relating to the chemical structures, substituents, derivatives, intermediates, syntheses, compositions, formulations, or methods of use of the disclosure, may be made without departing from the spirit and scope thereof.

For reasons of completeness, various aspects of the disclosure are set out in the following numbered clauses:
Clause 1. A composition comprising at least two sets of primers and at least two probes for detecting *Severe fever with thrombocytopenia syndrome virus* (SFTSV) in a sample obtained from a subject, wherein the composition comprises:
   a first set of primers and a first probe, wherein each primer comprises a region that corresponds to a portion of a L segment of the SFTSV, and further wherein the first set of primers comprise (i) a forward primer comprising or consisting of an oligonucleotide sequence of SEQ ID NO: 1 or a complement thereof, and (ii) a reverse primer comprising or consisting of an oligonucleotide sequence of SEQ ID NO: 2 and/or 3 or a complement thereof; and the first probe comprises or consists of an oligonucleotide sequence of SEQ ID NO: 4 and/or 5 or a complement thereof; and a second set of primers and a second probe, wherein each primer comprises a region that corresponds to an S segment of the SFTSV, and further wherein the second set of primers comprise (i) a forward primer comprising or consisting of an oligonucleotide sequence of SEQ ID NO: 6 or a complement thereof, and (ii) a reverse primer comprising or consisting of an oligonucleotide sequence of SEQ ID NO: 7 or a complement thereof; and the second probe comprises or consists of an oligonucleotide of SEQ ID NO: 8 or a complement thereof; and/or a third set of primers and a third probe, wherein each primer comprises a region that corresponds to an S segment of the SFTSV, and further wherein the third set of primers comprise (i) a forward primer comprising or consisting of an oligonucleotide sequence of SEQ ID NO: 9 or a complement thereof, and (ii) a reverse primer comprising or consisting of an oligonucleotide sequence of SEQ ID NO: 10 or a complement thereof; and the third probe comprises or consists of an oligonucleotide of SEQ ID NO: 11 and/or 12 or a complement thereof.
Clause 2. The composition of clause 1, wherein the composition comprises the first, second, and third sets of primers and probes.
Clause 3. The composition of clause 1 or 2 wherein: (a) in the first set of primers and the first probe: (i) the forward primer further comprises a detectable label; (ii) the reverse primer further comprises a detectable label; (iii) the first probe further comprises a detectable label; or (iv) any combination of (i)-(iii); (b) in the second sets of primers and the second probes: (i) the forward primer further comprises a detectable label; (ii) the reverse primer further comprises a detectable label; (iii) the second probe further comprises a detectable label; or (iv) any combination of (i)-(iii); (c) in the third set of primers and the third probe: (i) the forward primer further comprises a detectable label; (ii) the reverse primer further comprises a detectable label; (iii) the second probe further comprises a detectable label; or (iv) a combination of (i)-(iii); or (d) any combination of (a)-(c).
Clause 4. The composition of clause 3, wherein the first, second, and third probes comprise a detectable label.
Clause 5. The composition of clause 4, wherein the second and third probes comprise the same label.
Clause 6. The composition of clause 4 or 5, wherein the first probe comprises a different label than the second and third probes.
Clause 7. The composition of any one of clauses 3 to 6, wherein the first, second, and third probes comprise a quencher.
Clause 8. The composition of clause 7, wherein the quencher is located at the 3' end of the probe and the label is located at the 5' end of the probe.
Clause 9. The composition of any one of clauses 4 to 8, wherein the quencher is selected from tetramethylrhodamine (TAMRA) and black hole quencher.
Clause 10. The composition of any one of clauses 4 to 9, wherein the detectable label is a fluorophore.
Clause 11. The composition of clause 10, wherein the fluorophore is selected from carboxyfluorescein (FAM), tetrachlorofluorescein (TET), cyanine 5 (Cy5), and hexachlorofluorescein (HEX).
Clause 12. A kit for detecting SFTSV in sample obtained from a subject, the kit comprising: a) a first set of primers and a first probe, wherein each primer comprises a region that corresponds to a portion of a L segment of the SFTSV, and further wherein the first set of primers comprise (i) a forward primer comprising or consisting of an oligonucleotide sequence of SEQ ID NO: 1 or a complement thereof, and (ii) a reverse primer comprising or consisting of an oligonucleotide sequence of SEQ ID NO: 2 and/or 3 or a complement thereof; and the first probe comprises or consists of an oligonucleotide sequence of SEQ ID NO: 4 and/or 5 or a complement thereof; and b) a second set of primers and a second probe, wherein each primer comprises a region that corresponds to an S segment of the SFTSV, and further wherein the second set of primers comprise (i) a forward primer comprising or consisting of an oligonucleotide sequence of SEQ ID NO: 6 or a complement thereof, and (ii) a reverse primer comprising or consisting of an oligonucleotide sequence of SEQ ID NO: 7 or a complement thereof; and the second probe comprises or consists of an oligonucleotide of SEQ ID NO: 8 or a complement thereof; and/or c) a third set of primers and a third probe, wherein each primer comprises a region that corresponds to an S segment of the SFTSV, and further wherein the third set of primers comprise (i) a forward primer comprising or consisting of an oligonucleotide sequence of SEQ ID NO: 9 or a complement thereof, and (ii) a reverse primer comprising or consisting of an oligonucleotide sequence of SEQ ID NO: 10 or a complement thereof; and the third probe comprises or consists of an oligonucleotide of SEQ ID NO: 11 and/or 12 or a complement thereof.
Clause 13. The kit of clause 12, wherein the kit further comprises reagents and instructions for using the kit.
Clause 14. The kit of clause 13, wherein the reagents are selected from dNTPs, polymerases, nucleases, and buffers.
Clause 15. A method of detecting SFTSV in sample obtained from a subject, wherein the method comprises the steps of: a) performing a first amplification and detection reaction with a first set of primers and a first probe, wherein each primer comprises a region that corresponds to a portion of a L segment of the SFTSV, and further wherein the first set of primers comprise (i) a forward primer comprising or consisting of an oligonucleotide sequence of SEQ ID NO:1 or a complement thereof, and (ii) a reverse primer comprising or consisting of an oligonucleotide sequence of SEQ ID NO: 2 and/or 3 or a complement thereof; and the first probe comprises or consists of an oligonucleotide sequence of SEQ ID NO: 4 and/or 5 or a complement thereof, wherein the first amplification and detection reaction generates a first amplified product when an SFTSV L segment is present in the sample; and b) performing a second amplification and detection reaction with a second set of primers and a second probe, wherein each primer comprises a region that corresponds to an S segment of the SFTSV, and further wherein the second set of primers comprise (i) a forward primer comprising or consisting of an oligonucleotide sequence of SEQ ID NO: 6 or a complement thereof, and (ii) a reverse primer comprising or consisting of an oligonucleotide sequence of SEQ ID NO: 7 or a complement thereof; and the second probe comprises or consists of an oligonucleotide of SEQ ID NO: 8 or a complement thereof, wherein the second amplification and detection reaction generates a second amplified product when an SFTSV S segment is present in the sample; and/or c) performing a third amplification and detection reaction with a third set of primers and a third probe, wherein each primer comprises a region that corresponds to an S segment of the SFTSV, and further wherein the third set of primers comprise (i) a forward primer comprising or consisting of an oligonucleotide sequence of SEQ ID NO: 9 or a complement thereof, and (ii) a reverse primer comprising or consisting of an oligonucleotide sequence of SEQ ID NO: 10 or a complement thereof; and the third probe comprises or consists of an oligonucleotide of SEQ ID NO: 11 and/or 12 or a complement thereof, wherein the third amplification and detection reaction generates a third amplified product when an SFTSV S segment is present in the sample, and detecting the presence or absence of the first, second, and third amplified products, wherein the presence of at least the first amplified product and the second or third amplified products is indicative of the presence of SFTVS in the sample.
Clause 16. The method of clause 15, wherein the first, second and third amplification and detection reactions further comprise a reverse transcription step.
Clause 17. The method of clause 15 or 16, wherein the first, second and third amplification and detection reactions are real time PCR or real time reverse-transcriptase PCR reactions.
Clause 18. The method of any one of clauses 15 to 17, wherein the first, second and third amplification and detection reactions are quantitative real time PCR or real time reverse-transcriptase PCR reactions.
Clause 19. The method of any one of clauses 15 to 17, wherein the first, second and third amplification and detection reactions comprise a probe displacement and dequenching step.
Clause 20. The method of any one of clauses 15 to 19, wherein the first, second and third amplification and detection reactions are automated.
Clause 21. The method of any one of clauses 15 to 20, wherein the first, second and third amplification and detection reactions further comprise a sample processing step.
Clause 22. The method of clause 21, wherein the sample processing step is automated.
Clause 23. The method of clause 21 or 22, wherein the sample is selected from a blood sample, a plasma sample, and a serum sample.
Clause 24. The method of any one of clauses 15 to 23, wherein the subject is a human subject.
Clause 25. The method of any one of clauses 15 to 24, wherein the limit of detection for SFTVS is approximately one copy per sample.

### References

Acevedo, A. M., C. L. Perera, A. Vega, L. Ríos, L. Coronado, D. Relova, M. T. Frías, L. Ganges, J. I. Núñez and L. J. Pérez (2013). "A duplex SYBR Green I-based real-time RT-PCR assay for the simultaneous detection and differentiation of Massachusetts and non-Massachusetts serotypes of infectious bronchitis virus." Mol Cell Probes 27(5-6): 184-192.
Akagi, K., T. Miyazaki, K. Oshima, A. Umemura, S. Shimada, K. Morita, H. Senju, M. Tashiro, T. Takazono, T. Saijo, S. Kurihara, M. Sekino, K. Yamamoto, Y. Imamura, K. Izumikawa, K. Yanagihara, A. Uda, S. Morikawa, T. Yoshikawa, T. Kurosu, M. Shimojima, M. Saijo and H. Mukae (2020). "Detection of viral RNA in diverse body fluids in an SFTS patient with encephalopathy, gastrointestinal bleeding and pneumonia: a case report and literature review." BMC Infect Dis 20(1): 281.
Anderson, M., A. Hodges, K. C. Luk, A. Olivo, K. Forberg, T. V. Meyer, C. Strobel, M. Kim, D. Toolsie, N. M. Moore, Y. Goldstein, M. Rodgers, D. Lucic and G. Cloherty (2024). "Development and Validation of Three Automated High-Throughput Molecular Tests to Detect Monkeypox Virus Infections." J Infect Dis 229(Supplement_2): S137-s143.
Averhoff, F., M. Berg, M. Rodgers, S. Osmanov, X. Luo, M. Anderson, T. Meyer, A. Landay, A. Gamkrelidze, E. G. Kallas, K. Ciuoderis, J. P. Hernandez, J. H. Henry, J. Osorio, J. Lindo, J. Deshommes, J. Anzinger, J. Manasa, M. Alkashvili, M. Souleyman, P. Kaleebu, R. Correa-Oliveira, S. Solomon, T. de Olivera, Y. Suputtamongkol and G. Cloherty (2022). "The Abbott Pandemic Defense Coalition: a unique multisector approach adds to global pandemic preparedness efforts." Int. J. Infect. Dis. 117: 356-360.
Ayres, D. L., M. P. Cummings, G. Baele, A. E. Darling, P. O. Lewis, D. L. Swofford, J. P. Huelsenbeck, P. Lemey, A. Rambaut and M. A. Suchard (2019). "BEAGLE 3: Improved Performance, Scaling, and Usability for a High-Performance Computing Library for Statistical Phylogenetics." Syst Biol 68(6): 1052-1061.
Bustin, S. A. and T. Nolan (2004). "Pitfalls of quantitative real-time reverse-transcription polymerase chain reaction." J Biomol Tech 15(3): 155-166.
Casel, M. A., S. J. Park and Y. K. Choi (2021). "Severe fever with thrombocytopenia syndrome virus: emerging novel phlebovirus and their control strategy." Experimental & Molecular Medicine 53(5): 713-722.
Cleveland, W. S. and S. J. Devlin (1988). "Locally Weighted Regression: An Approach to Regression Analysis by Local Fitting." Journal of the American Statistical Association 83(403): 596-610.
Du, Y., N. Cheng, Y. Li, H. Wang, A. You, J. Su, Y. Nie, H. Ma, B. Xu and X. Huang (2019). "Seroprevalance of antibodies specific for severe fever with thrombocytopenia syndrome virus and the discovery of asymptomatic infections in Henan Province, China." PLoS Negl Trop Dis 13(11): e0007242.
Gu, Z., L. Gu, R. Eils, M. Schlesner and B. Brors (2014). "circlize implements and enhances circular visualization in R." Bioinformatics 30(19): 2811-2812.
Han, M. A., C. M. Kim, D. M. Kim, N. R. Yun, S. W. Park, M. G. Han and W. J. Lee (2018). "Seroprevalence of Severe Fever with Thrombocytopenia Syndrome Virus Antibodies in Rural Areas, South Korea." Emerg Infect Dis 24(5): 872-874.
Heo, S. T., J. R. Yoo, K. H. Lee and K. S. Ko (2015). "The First Case of Non-retrospective Clinical Identification of Severe Fever with Thrombocytopenia Syndrome Patient in 2013 in South Korea." jbv 45(2): 155-158.
Hill, V. and G. Baele (2019). "Bayesian Estimation of Past Population Dynamics in BEAST 1.10 Using the Skygrid Coalescent Model." Molecular Biology and Evolution 36(11): 2620-2628.
Huang, D., Y. Jiang, X. Liu, B. Wang, J. Shi, Z. Su, H. Wang, T. Wang, S. Tang, H. Liu, Z. Hu, F. Deng and S. Shen (2017). "A Cluster of Symptomatic and Asymptomatic Infections of Severe Fever with Thrombocytopenia Syndrome Caused by Person-to-Person Transmission." Am J Trop Med Hyg 97(2): 396-402.
Ishijima, K., T. Phichitraslip, N. Naimon, P. Ploypichai, B. Kriebkajon, T. Chinarak, J. Sridaphan, A. Kritiyakan, N. Prasertsincharoen, S. Jittapalapong, K. Tangcham, W. Rerkamnuaychoke, Y. Kuroda, M. Taira, K. Tatemoto, E. Park, M. Virhuez-Mendoza, Y. Inoue, M. Harada, T. Yamamoto, A. Nishino, A. Matsuu and K. Maeda (2023). "High Seroprevalence of Severe Fever with Thrombocytopenia Syndrome Virus Infection among the Dog Population in Thailand." Viruses 15(12).
Jacoby, W. G. (2000). "Loess:: a nonparametric, graphical tool for depicting relationships between variables." Electoral Studies 19(4): 577-613.
Jalal, S., S. Y. Hwang, C.-M. Kim, D.-M. Kim, N. R. Yun, J.-W. Seo, D. Young Kim, S. I. Jung, U. J. Kim, S. E. Kim, H. a. Kim, E. S. Kim, J. Hur, Y. K. Kim, H. W. Jeong, J. Y. Heo, D. S. Jung, J. Kim, S. H. Park, Y. G. Kwak, S. Lee, S. Lim and S. H. Lee (2021). "Comparison of RT-PCR, RT-nested PCRs, and real-time PCR for diagnosis of severe fever with thrombocytopenia syndrome: a prospective study." Scientific Reports 11(1): 16764.
Jang, W. S., D. H. Lim, Y. L. Choe, J. Nam, K. C. Moon, C. Kim, M. Choi, I. Park, D. W. Park and C. S. Lim (2022). "Developing a multiplex loop-mediated isothermal amplification assay (LAMP) to determine severe fever with thrombocytopenia syndrome (SFTS) and scrub typhus." PLoS One 17(2): e0262302.
Kim, E. H. and S. J. Park (2023). "Emerging Tick-Borne Dabie bandavirus: Virology, Epidemiology, and Prevention." Microorganisms 11(9).
Kim, K. H., J. Yi, G. Kim, S. J. Choi, K. I. Jun, N. H. Kim, P. G. Choe, N. J. Kim, J. K. Lee and M. D. Oh (2013). "Severe fever with thrombocytopenia syndrome, South Korea, 2012." Emerg Infect Dis 19(11): 1892-1894.
Kimura, T., A. Fukuma, M. Shimojima, Y. Yamashita, F. Mizota, M. Yamashita, Y. Otsuka, M. Kan, S. Fukushi, H. Tani, S. Taniguchi, M. Ogata, T. Kurosu, S. Morikawa, M. Saijo and H. Shinomiya (2018). "Seroprevalence of severe fever with thrombocytopenia syndrome (SFTS) virus antibodies in humans and animals in Ehime prefecture, Japan, an endemic region of SFTS." J Infect Chemother 24(10): 802-806.
Lemey, P., S. L. Hong, V. Hill, G. Baele, C. Poletto, V. Colizza, Á. O'Toole, J. T. McCrone, K. G. Andersen, M. Worobey, M. I. Nelson, A. Rambaut and M. A. Suchard (2020). "Accommodating individual travel history and unsampled diversity in Bayesian phylogeographic inference of SARS-CoV-2." Nat. Commun. 11(1): 5110.
Lemey, P., A. Rambaut, A. J. Drummond and M. A. Suchard (2009). "Bayesian Phylogeography Finds Its Roots." PLOS Computational Biology 5(9): e1000520.
Lemey, P., N. Ruktanonchai, S. L. Hong, V. Colizza, C. Poletto, F. Van den Broeck, M. S. Gill, X. Ji, A. Levasseur, B. B. Oude Munnink, M. Koopmans, A. Sadilek, S. Lai, A. J. Tatem, G. Baele, M. A. Suchard and S. Dellicour (2021). "Untangling introductions and persistence in COVID-19 resurgence in Europe." Nature 595(7869): 713-717.
Li, P., Z. D. Tong, K. F. Li, A. Tang, Y. X. Dai and J. B. Yan (2017). "Seroprevalence of severe fever with thrombocytopenia syndrome virus in China: A systematic review and meta-analysis." PLoS One 12(4): e0175592.
Li, S., Y. Li, Q. Wang, X. Yu, M. Liu, H. Xie, L. Qian, L. Ye, Z. Yang, J. Zhang, H. Zhu and W. Zhang (2018). "Multiple organ involvement in severe fever with thrombocytopenia syndrome: an immunohistochemical finding in a fatal case." Virol J 15(1): 97.
Liu, J. W., L. Zhao, L. M. Luo, M. M. Liu, Y. Sun, X. Su and X. J. Yu (2016). "Molecular Evolution and Spatial Transmission of Severe Fever with Thrombocytopenia Syndrome Virus Based on Complete Genome Sequences." PLoS One 11(3): e0151677.
Liu, K., H. Zhou, R.-X. Sun, H.-W. Yao, Y. Li, L.-P. Wang, M. Di, X.-L. Li, Y. Yang, G. C. Gray, N. Cui, W.-W. Yin, L.-Q. Fang, H.-J. Yu and W.-C. Cao (2015). "A National Assessment of the Epidemiology of Severe Fever with Thrombocytopenia Syndrome, China." Scientific Reports 5(1): 9679.
Liu, Q., B. He, S. Y. Huang, F. Wei and X. Q. Zhu (2014). "Severe fever with thrombocytopenia syndrome, an emerging tick-borne zoonosis." Lancet Infect Dis 14(8): 763-772.
Luo, N., M. Li, M. Xu, C. Shi, X. Shi, R. Ni, Y. Chen, L. Zheng, Y. Tu, D. Hu, C. Yu, Q. Li and Y. Lu (2023). "Research Progress of Fever with Thrombocytopenia Syndrome." Intensive Care Res: 1-10.
Nguyen, L. T., H. A. Schmidt, A. von Haeseler and B. Q. Minh (2015). "IQ-TREE: a fast and effective stochastic algorithm for estimating maximum-likelihood phylogenies." Mol Biol Evol 32(1): 268-274.
Park, S. W., B. G. Song, E. H. Shin, S. M. Yun, M. G. Han, M. Y. Park, C. Park and J. Ryou (2014). "Prevalence of severe fever with thrombocytopenia syndrome virus in Haemaphysalis longicornis ticks in South Korea." Ticks Tick Borne Dis 5(6): 975-977.
Peng, S. H., S. L. Yang, S. E. Tang, T. C. Wang, T. C. Hsu, C. L. Su, M. Y. Chen, M. Shimojima, T. Yoshikawa and P. Y. Shu (2020). "Human Case of Severe Fever with Thrombocytopenia Syndrome Virus Infection, Taiwan, 2019." Emerg Infect Dis 26(7): 1612-1614.
Pérez, L. J., G. Baele, S. L. Hong, G. A. Cloherty and M. G. Berg (2024). "Ecological Changes Exacerbating the Spread of Invasive Ticks has Driven the Dispersal of Severe Fever with Thrombocytopenia Syndrome Virus Throughout Southeast Asia." Mol Biol Evol 41(8).
Perez, L. J., G. A. Cloherty and M. G. Berg (2021). "Understanding the Genetic Diversity of Picobirnavirus: A Classification Update Based on Phylogenetic and Pairwise Sequence Comparison Approaches." Viruses 13(8).
Pérez, L. J., S. Lanka, V. J. DeShambo, R. L. Fredrickson and C. W. Maddox (2020). "A Validated Multiplex Real-Time PCR Assay for the Diagnosis of Infectious Leptospira spp.: A Novel Assay for the Detection and Differentiation of Strains From Both Pathogenic Groups I and II." Front Microbiol 11: 457.
Rattanakomol, P., S. Khongwichit, P. Linsuwanon, K. H. Lee, S. Vongpunsawad and Y. Poovorawan (2022). "Severe Fever with Thrombocytopenia Syndrome Virus Infection, Thailand, 2019-2020." Emerg Infect Dis 28(12): 2572-2574.
Redman, S. A., L. J. Perez, K. Forberg, K. Francis, J. P. Walker, T. K. Thompson, H. Phillips, G. A. Cloherty, M. G. Berg and J. J. Anzinger (2024). "Dengue Virus Serotype 3 Origins and Genetic Dynamics, Jamaica." Emerg Infect Dis 30(10): 2149-2154.
Rios, L., C. L. Perera, L. Coronado, D. Relova, A. M. Álvarez, L. Ganges, H. Diaz de Arce, J. I. Núñez and L. J. Pérez (2018). "Multi-Target Strategy for Pan/Foot- and-Mouth Disease Virus (FMDV) Detection: A Combination of Sequences Analysis, in Silico Predictions and Laboratory Diagnostic Evaluation." Front Vet Sci 5: 160.
Rozewicki, J., S. Li, K. M. Amada, D. M. Standley and K. Katoh (2019). "MAFFT-DASH: integrated protein sequence and structural alignment." Nucleic Acids Research 47(W1): W5-W10.
Saba Villarroel, P. M., T. Chaiphongpachara, E. Nurtop, S. Laojun, T. Pangpoo-Nga, T. Songhong, D. Supungul, C. Baronti, L. Thirion, P. Leaungwutiwong, X. de Lamballerie, D. Missé and S. Wichit (2024). "Seroprevalence study in humans and molecular detection in Rhipicephalus sanguineus ticks of severe fever with thrombocytopenia syndrome virus in Thailand." Sci Rep 14(1): 13397.
Sato, Y., H. Mekata, P. E. Sudaryatma, Y. Kirino, S. Yamamoto, S. Ando, T. Sugimoto and T. Okabayashi (2021). "Isolation of Severe Fever with Thrombocytopenia Syndrome Virus from Various Tick Species in Area with Human Severe Fever with Thrombocytopenia Syndrome Cases." Vector Borne Zoonotic Dis 21(5): 378-384.
Shaikh, N., P. Swali and R. Houben (2023). "Asymptomatic but infectious - The silent driver of pathogen transmission. A pragmatic review." Epidemics 44: 100704.
Sun, Y., M. Liang, J. Qu, C. Jin, Q. Zhang, J. Li, X. Jiang, Q. Wang, J. Lu, W. Gu, S. Zhang, C. Li, X. Wang, F. Zhan, W. Yao, Z. Bi, S. Wang and D. Li (2012). "Early diagnosis of novel SFTS bunyavirus infection by quantitative real-time RT-PCR assay." J Clin Virol 53(1): 48-53.
Takahashi, T., K. Maeda, T. Suzuki, A. Ishido, T. Shigeoka, T. Tominaga, T. Kamei, M. Honda, D. Ninomiya, T. Sakai, T. Senba, S. Kaneyuki, S. Sakaguchi, A. Satoh, T. Hosokawa, Y. Kawabe, S. Kurihara, K. Izumikawa, S. Kohno, T. Azuma, K. Suemori, M. Yasukawa, T. Mizutani, T. Omatsu, Y. Katayama, M. Miyahara, M. Ijuin, K. Doi, M. Okuda, K. Umeki, T. Saito, K. Fukushima, K. Nakajima, T. Yoshikawa, H. Tani, S. Fukushi, A. Fukuma, M. Ogata, M. Shimojima, N. Nakajima, N. Nagata, H. Katano, H. Fukumoto, Y. Sato, H. Hasegawa, T. Yamagishi, K. Oishi, I. Kurane, S. Morikawa and M. Saijo (2013). "The First Identification and Retrospective Study of Severe Fever With Thrombocytopenia Syndrome in Japan." The Journal of Infectious Diseases 209(6): 816-827.
Tran, X. C., S. H. Kim, J. E. Lee, S. H. Kim, S. Y. Kang, N. D. Binh, P. V. Duc, P. T. K. Phuong, N. T. P. Thao, W. Lee, J. Y. Bae, M. S. Park, M. Kim, J. R. Yoo, S. T. Heo, K. H. An, J. M. Kim, N. H. Cho, S. H. Kee and K. H. Lee (2022). "Serological Evidence of Severe Fever with Thrombocytopenia Syndrome Virus and IgM Positivity Were Identified in Healthy Residents in Vietnam." Viruses 14(10).
Tran, X. C., Y. Yun, L. Van An, S. H. Kim, N. T. P. Thao, P. K. C. Man, J. R. Yoo, S. T. Heo, N. H. Cho and K. H. Lee (2019). "Endemic Severe Fever with Thrombocytopenia Syndrome, Vietnam." Emerg Infect Dis 25(5): 1029-1031.
Wang, Y., B. Deng, J. Zhang, W. Cui, W. Yao and P. Liu (2014). "Person-to-person asymptomatic infection of severe fever with thrombocytopenia syndrome virus through blood contact." Intern Med 53(8): 903-906.
Win, A. M., Y. T. H. Nguyen, Y. Kim, N. Y. Ha, J. G. Kang, H. Kim, B. San, O. Kyaw, W. W. Htike, D. O. Choi, K. H. Lee and N. H. Cho (2020). "Genotypic Heterogeneity of Orientia tsutsugamushi in Scrub Typhus Patients and Thrombocytopenia Syndrome Co-infection, Myanmar." Emerg Infect Dis 26(8): 1878-1881.
Xu, A. L., H. Xue, Y. Li, X. Wang, J. X. Zheng, F. Y. Shi, Q. X. Cui, Y. Lu, D. J. Cun and L. H. Li (2024). "Comprehensive meta-analysis of severe fever with thrombocytopenia syndrome virus infections in humans, vertebrate hosts and questing ticks." Parasit Vectors 17(1): 265.
Yu, X. J., M. F. Liang, S. Y. Zhang, Y. Liu, J. D. Li, Y. L. Sun, L. Zhang, Q. F. Zhang, V. L. Popov, C. Li, J. Qu, Q. Li, Y. P. Zhang, R. Hai, W. Wu, Q. Wang, F. X. Zhan, X. J. Wang, B. Kan, S. W. Wang, K. L. Wan, H. Q. Jing, J. X. Lu, W. W. Yin, H. Zhou, X. H. Guan, J. F. Liu, Z. Q. Bi, G. H. Liu, J. Ren, H. Wang, Z. Zhao, J. D. Song, J. R. He, T. Wan, J. S. Zhang, X. P. Fu, L. N. Sun, X. P. Dong, Z. J. Feng, W. Z. Yang, T. Hong, Y. Zhang, D. H. Walker, Y. Wang and D. X. Li (2011). "Fever with thrombocytopenia associated with a novel bunyavirus in China." N Engl J Med 364(16): 1523-1532.
Yun, S.-M., S.-J. Park, Y.-I. Kim, S.-W. Park, M.-A. Yu, H.-I. Kwon, E.-H. Kim, K.-M. Yu, H. W. Jeong, J. Ryou, W.-J. Lee, Y. Jee, J.-Y. Lee and Y. K. Choi (2020). "Genetic and pathogenic diversity of severe fever with thrombocytopenia syndrome virus (SFTSV) in South Korea." JCI Insight 5(2).
Zhou, J., Q. Wang, L. Zhu, S. Li, W. Li, Y. Fu and X. Cheng (2020). "Development and evaluation of a rapid detection assay for severe fever with thrombocytopenia syndrome virus based on reverse-transcription recombinase polymerase amplification." Mol Cell Probes 52: 101580.
Zohaib, A., J. Zhang, M. Saqib, M. A. Athar, M. H. Hussain, J. Chen, A. U. Sial, M. H. Tayyab, M. Batool, S. Khan, Y. Luo, C. Waruhiu, Z. Taj, Z. Hayder, R. Ahmed, A. B. Siddique, X. Yang, M. A. Qureshi, I. U. Ujjan, A. Lail, I. Khan, R. Sajjad Ur, T. Zhang, F. Deng, Z. Shi and S. Shen (2020). "Serologic Evidence of Severe Fever with Thrombocytopenia Syndrome Virus and Related Viruses in Pakistan." Emerg Infect Dis 26(7): 1513-1516.

## Claims

1. A composition comprising at least two sets of primers and at least two probes for detecting *Severe fever with thrombocytopenia syndrome virus* (SFTSV) in a sample obtained from a subject, wherein the composition comprises:
a) a first set of primers and a first probe, wherein each primer comprises a region that corresponds to a portion of a L segment of the SFTSV, and further wherein the first set of primers comprise (i) a forward primer comprising or consisting of an oligonucleotide sequence of SEQ ID NO:1 or a complement thereof, and (ii) a reverse primer comprising or consisting of an oligonucleotide sequence of SEQ ID NO: 2 and/or 3 or a complement thereof; and the first probe comprises or consists of an oligonucleotide sequence of SEQ ID NO: 4 and/or 5 or a complement thereof; and
b) a second set of primers and a second probe, wherein each primer comprises a region that corresponds to an S segment of the SFTSV, and further wherein the second set of primers comprise (i) a forward primer comprising or consisting of an oligonucleotide sequence of SEQ ID NO: 6 or a complement thereof, and (ii) a reverse primer comprising or consisting of an oligonucleotide sequence of SEQ ID NO: 7 or a complement thereof; and the second probe comprises or consists of an oligonucleotide of SEQ ID NO: 8 or a complement thereof; and/or
c) a third set of primers and a third probe, wherein each primer comprises a region that corresponds to an S segment of the SFTSV, and further wherein the third set of primers comprise (i) a forward primer comprising or consisting of an oligonucleotide sequence of SEQ ID NO: 9 or a complement thereof, and (ii) a reverse primer comprising or consisting of an oligonucleotide sequence of SEQ ID NO: 10 or a complement thereof; and the third probe comprises or consists of an oligonucleotide of SEQ ID NO: 11 and/or 12 or a complement thereof.

2. The composition of claim 1, wherein the composition comprises the first, second, and third sets of primers and probes.

3. The composition of claim 1 or 2 wherein: (a) in the first set of primers and the first probe: (i) the forward primer further comprises a detectable label; (ii) the reverse primer further comprises a detectable label; (iii) the first probe further comprises a detectable label; or (iv) a combination of (i)-(iii); (b) in the second sets of primers and the second probes: (i) the forward primer further comprises a detectable label; (ii) the reverse primer further comprises a detectable label; (iii) the second probe further comprises a detectable label; or (iv) a combination of (i)-(iii); (c) in the third set of primers and the third probe: (i) the forward primer further comprises a detectable label; (ii) the reverse primer further comprises a detectable label; (iii) the second probe further comprises a detectable label; or (iv) a combination of (i)-(iii); or (d) any combination of (a)-(c).

4. The composition of claim 3, wherein the first, second, and third probes comprise a detectable label; optionally wherein the second and third probes comprise the same label.

5. The composition of claim 4, wherein the first probe comprises a different label than the second and third probes.

6. The composition of any one of claims 3 to 5, wherein the first, second, and third probes comprise a quencher; optionally wherein the quencher is located at the 3' end of the probe and the label is located at the 5' end of the probe.

7. The composition of claim 6, wherein the quencher is selected from tetramethylrhodamine (TAMRA) and black hole quencher.

8. The composition of any one of claims 4 to 7, wherein the detectable label is a fluorophore; optionally wherein the fluorophore is selected from carboxyfluorescein (FAM), tetrachlorofluorescein (TET), cyanine 5 (Cy5), and hexachlorofluorescein (HEX).

9. A kit for detecting SFTSV in sample obtained from a subject, the kit comprising:
a) a first set of primers and a first probe, wherein each primer comprises a region that corresponds to a portion of a L segment of the SFTSV, and further wherein the first set of primers comprise (i) a forward primer comprising or consisting of an oligonucleotide sequence of SEQ ID NO:1 or a complement thereof, and (ii) a reverse primer comprising or consisting of an oligonucleotide sequence of SEQ ID NO: 2 and/or 3 or a complement thereof; and the first probe comprises or consists of an oligonucleotide sequence of SEQ ID NO: 4 and/or 5 or a complement thereof; and
b) a second set of primers and a second probe, wherein each primer comprises a region that corresponds to an S segment of the SFTSV, and further wherein the second set of primers comprise (i) a forward primer comprising or consisting of an oligonucleotide sequence of SEQ ID NO: 6 or a complement thereof, and (ii) a reverse primer comprising or consisting of an oligonucleotide sequence of SEQ ID NO:7 or a complement thereof; and the second probe comprises or consists of an oligonucleotide of SEQ ID NO: 8 or a complement thereof; and/or
c) a third set of primers and a third probe, wherein each primer comprises a region that corresponds to an S segment of the SFTSV, and further wherein the third set of primers comprise (i) a forward primer comprising or consisting of an oligonucleotide sequence of SEQ ID NO: 9 or a complement thereof, and (ii) a reverse primer comprising or consisting of an oligonucleotide sequence of SEQ ID NO: 10 or a complement thereof; and the third probe comprises or consists of an oligonucleotide of SEQ ID NO: 11 and/or 12 or a complement thereof.

10. The kit of claim 9, wherein the kit further comprises reagents and instructions for using the kit; optionally wherein the reagents are selected from dNTPs, polymerases, and buffers.

11. A method of detecting SFTSV in sample obtained from a subject, wherein the method comprises the steps of:
a) performing a first amplification and detection reaction with a first set of primers and a first probe, wherein each primer comprises a region that corresponds to a portion of a L segment of the SFTSV, and further wherein the first set of primers comprise (i) a forward primer comprising or consisting of an oligonucleotide sequence of SEQ ID NO: 1 or a complement thereof, and (ii) a reverse primer comprising or consisting of an oligonucleotide sequence of SEQ ID NO: 2 and/or 3 or a complement thereof; and the first probe comprises or consists of an oligonucleotide sequence of SEQ ID NO: 4 and/or 5 or a complement thereof, wherein the first amplification and detection reaction generates a first amplified product when an SFTSV L segment is present in the sample; and
b) performing a second amplification and detection reaction with a second set of primers and a second probe, wherein each primer comprises a region that corresponds to an S segment of the SFTSV, and further wherein the second set of primers comprise (i) a forward primer comprising or consisting of an oligonucleotide sequence of SEQ ID NO: 6 or a complement thereof, and (ii) a reverse primer comprising or consisting of an oligonucleotide sequence of SEQ ID NO: 7 or a complement thereof; and the second probe comprises or consists of an oligonucleotide of SEQ ID NO: 8 or a complement thereof, wherein the second amplification and detection reaction generates a second amplified product when an SFTSV S segment is present in the sample; and/or c) performing a third amplification and detection reaction with a third set of primers and a third probe, wherein each primer comprises a region that corresponds to an S segment of the SFTSV, and further wherein the third set of primers comprise (i) a forward primer comprising or consisting of an oligonucleotide sequence of SEQ ID NO: 9 or a complement thereof, and (ii) a reverse primer comprising or consisting of an oligonucleotide sequence of SEQ ID NO: 10 or a complement thereof; and the third probe comprises or consists of an oligonucleotide of SEQ ID NO: 11 and/or 12 or a complement thereof, wherein the third amplification and detection reaction generates a third amplified product when an SFTSV S segment is present in the sample, and detecting the presence or absence of the first, second, and third amplified products, wherein the presence of at least the first amplified product and the second or third amplified products is indicative of the presence of SFTVS in the sample.

12. The method of claim 11, wherein:
(a) the first, second and third amplification and detection reactions further comprise a reverse transcription step; and/or
(b) the first, second and third amplification and detection reactions are real time PCR or real time reverse-transcriptase PCR reactions.

13. The method of claim 11 or 12, wherein:
(a) the first, second and third amplification and detection reactions are quantitative real time PCR or real time reverse-transcriptase PCR reactions; or
(b) the first, second and third amplification and detection reactions comprise a probe displacement and dequenching step.

14. The method of any one of claims 11 to 13, wherein:
(a) the first, second and third amplification and detection reactions are automated; and/or
(b) the first, second and third amplification and detection reactions further comprise a sample processing step; optionally wherein the sample processing step is automated.

15. The method of any one of claims 11 to 14, wherein:
(a) the sample is selected from a blood sample, a plasma sample, and a serum sample; and/or
(b) the subject is a human subject; and/or
(c) the limit of detection for SFTVS is approximately one copy per sample.
